# EUROPEAN PATENT SPECIFICATION

(11) **EP 3 517 964 B1**
(45) Date of publication and mention of the grant of the patent: **13.04.2022**
(21) Application number: 18210390.3
(22) Date of filing: 20.03.2015
(51) Int. Cl.: G01N 33/68

(54) **NEW MARKERS FOR THE ASSESSMENT OF THE RISK FOR DEVELOPMENT OF A CARDIOVASCULAR DISORDER**
NEUE MARKER ZUR BEURTEILUNG DES RISIKOS DER ENTWICKLUNG EINER HERZ-KREISLAUF-ERKRANKUNG
NOUVEAUX MARQUEURS POUR L'ÉVALUATION DU RISQUE DE DÉVELOPPEMENT DE TROUBLE CARDIOVASCULAIRE

(30) Priority: 21.03.2014 EP 14305406; 30.09.2014 EP 14306528
(43) Date of publication of application: 31.07.2019
(62) Divisional of application: 15710817.6
(73) Proprietor: Sanofi-Aventis Deutschland GmbH, 65929 Frankfurt (DE); McMaster University, Hamilton, Ontario L8S 4L8 (CA)
(72) Inventor: HESS, Sybille, 65926 Frankfurt am Main (DE); GERSTEIN, Hertzel, Hamilton, Ontario L8S 4A8 (CA); PARE, Guillaume, Oakville, Ontario L6H 2B5 (CA); MCQUEEN, Matthew, Burlington, Ontario L7P 3A8 (CA)
(74) Representative: Weickmann & Weickmann PartmbB

(56) References cited:
- WO-A1-2010/048346
- WO-A1-2013/163345
- WO-A2-2015/185672
- US-A1- 2013 085 079
- MATTIA A E VALENTE ET AL: "Urinary Proteins in Heart Failure", PROGRESS IN CARDIOVASCULAR DISEASES, vol. 55, no. 1, 1 July 2012 (2012-07-01), pages 44-55, XP028449268, ISSN: 0033-0620, DOI: 10.1016/J.PCAD.2012.04.009 [retrieved on 2012-05-01]
- HILLIS GRAHAM S ET AL: "The relative and combined ability of high-sensitivity cardiac troponin T and N-terminal pro-B-type natriuretic peptide to predict cardiovascular events and death in patients with type 2 diabetes.", DIABETES CARE JAN 2014, vol. 37, no. 1, January 2014 (2014-01), pages 295-303, XP009179019, ISSN: 1935-5548

## Description

The present invention relates to a method for assessing the risk for development of a cardiovascular disorder in a subject. It discloses the use of at least one marker selected from the group consisting of T Trefoil Factor 3, alpha-2-Macroglobulin, Vascular Endothelial Growth Factor D, Tamm-Horsfall Urine Glycoprotein, Kidney-Injury-Molecule-1, Hepatocyte Growth Factor Receptor, Neuropilin-1, Insulin-like Growth Factor Binding Protein 4, and alpha-Glutathione-S-Transferase in the assessment of the risk for development of a cardiovascular disorder in a subject.

Cardiovascular (CV) disease is the leading cause of death world-wide. Early assessment of cardiovascular disease in patients at risk of developing a cardiovascular disease is important for the development or modification of therapeutic strategies and patient's lifestyles.

One group of patients at risk of development of a cardiovascular disease consists of patients suffering from diabetes. The risk of developing a cardiovascular disease such as myocardial infarction accounts for about 50% all deaths in this patient group. For patients with type 2 diabetes the incidence of a cardiovascular disease is increased such that about 2 out of 3 patients die from a cardiovascular disease. Particularly for patients with type 2 diabetes the existing methods of risk prediction such as, e.g., biochemical markers are not significant.

A number of risk markers for the development of cardiovascular diseases are known:
The B-type natriuretic peptide (BNP) (also termed Brain natriuretic peptide) is a protein with 32 amino acids. It is secreted by the ventricles of the heart in response to excessive stretching of heart muscle cells. BNP is secreted as propeptide along with 76 amino acid long N-terminal fragment (NT-pro BNP; Swiss Prot Number P16860) which is biologically inactive. The biological half life of NT-pro BNP is higher than that of BNP, which makes NT-pro BNP an attractive diagnostic target. Increasing NT-pro BNP plasma levels (together with MCP-1 and Galectin-3) have been found to be associated with a greater incidence of CV events (Tunon J, Am J Cardiol, 2013). Furthermore, NT-pro BNP was identified as predictor of total mortality and CV morality in elderly (Muscari A, Int J Clin Pract, 2013). NT-pro BNP (and hs-cTnT) was also found to improve the accuracy with which the risk of CV events or death can be estimated in patients with type 2 diabetes (Hillis et al. 2013, Diabetes Care 37(1):295-303). NT-pro BNP (+hsTNT) was further found to predict mortality in stable coronary artery disease (Giannitsis E, Clin Chem Lab Med, 2013). Inclusion of NT-pro BNP markedly improved heart failure (HF) risk prediction of Framingham, Health ABC, and ARIC risk scores by 18%, 12%, and 13%. (Agarwal SK, Circ Heart Fail, 2012).

Peroxiredoxin-4 (Uniprot Number Q13162) is a secretable and stable isoform of the peroxiredoxin (Prx) family of antioxidant peroxidases which consists of 6 members. Due to its antioxidant activity, it is a protector against oxidative stress. Peroxiredoxin-4 is involved in the activation of the transcription factor NF-kappaB. It has been shown that levated serum level are associated with a significant higher risk of incident cardiovascular events and cardiovascular mortality and all-cause mortality in patients without history of cardiovascular disease (Abbasi A, JAHA, 2012). Moreover, high levels of Peroxiredoxin-4 have been observed in patients with type 2 diabetes and peripheral atherosclerosis disease (PAD) (Eter EI, Cell Stress Chaperones, 2013).

Osteoprotegerin (OPG; Swiss Prot Number O00300) is encoded by the TNFRSF11B gene. OPG is a cytokine receptor and a member of the tumor necrosis factor (TNF) receptor superfamily. OPG mainly binds to 2 ligands, i.e. RANKL (receptor activator of nuclear factor kappa B ligand) and TRAIL (tumor necrosis factor-related apoptosis-induced ligand). Upon binding to the former ligand, OPG inhibits activity of osteoclasts and promotes their apoptosis. OPG and RANKL are regulators of mineral metabolism in bone and vascular tissues and are markers for atherosclerosis. OPG can be considered as a "vascular calcification" marker. Binding of OPG with TRAIL prevents apoptosis of tumor cells. It has been shown that OPG improves risk prediction of traditional cardiovascular risk factors (Mogelvang R, Heart, 2012). It was further shown to be an independent predictor of cardiovascular complications in type 1 diabetics (Gordin D, Diab Care, 2013). Moreover, high level of OPG (+ 8 other markers) were shown to be associated with lower ankle-brachial index (ABI) (Ye Z, Am J Hypertension, 2013). In patients with older age an increase of OPG level was observed (Fontes JD, Atherosclerosis, 2013). OPG (and Lp-PLA2) were found to be elevated in patients with carotid artery stenosis (Musialek P, J Clin Neurol, 2013). OPG gene polymorphism was found to be associated with increased risk of cardiovascular disease in type 2 diabetics (Guo C, Genet. Mol Biol, 2013). Finally, OPG (and OPN) levels were found to be positively associated with arterial stiffness, and with the extent of CAD (Tousoulis, int J Cardiol, 2013).

Apolipoprotein B (ApoB; Swiss Prot Number P04114) is the primary apolipoprotein of chylomicrons and low-density lipoproteins (LDL). ApoB on the LDL particle acts as a ligand for LDL receptors in various cells throughout the body. Through a mechanism that is not fully understood, high levels of ApoB can lead to plaques that cause vascular disease (atherosclerosis), leading to heart disease. There is considerable evidence that levels of ApoB are a better indicator of heart disease risk than total cholesterol or LDL. ApoB was shown to be a more accurate marker of cardiovascular risk than non-HDL-C (Sniderman AD, Atherosclerosis, 2012). Apo B (and the Apolipoprotein B/Apolipoprotein (a)-I ratio) were shown to be associated with carotid intima-media thickness (Huang F, PLOSONE, 2013). Moreover, elevated ApoB serum levels have been shown to strongly predict early cardiovascular events (Gigante B, Heart, 212). ApoB was shown to be associated with increased risk of MetS incidence (Koskinen J, Eur J Prev Cardiol.2012). Finally, the ApoB/ApoA1 ratio was superior to any of the cholesterol ratios for estimation of the risk of acute myocardial infarction (McQueen MJ, Lancet, 2008).

Angiopoietin-2 (Ang-2; SwissProt Number 015123) is a protein that is encoded by the ANGPT2 gene in humans. ANG-2 belongs to the angiopoietin family which comprises 4 vascular growth factors that play a role in angiogenesis. Angiopoietin cytokines are involved with controlling microvascular permeability and allow the smooth muscle cells to cover the vessels making vasodilation and vasoconstriction possible. Angiopoietin-2 promotes cell death and disrupts vascularisation. Circulating Ang-2 (and its receptor sTie-2) were determined has heritable traits and were associated with cardiovascular disease risk factors, including metabolic syndrome (Lieb W, Circ CV Genet, 2010). High levels of ANG-2 were found to be associated with a greater risk of all-cause and cardiovascular mortality (Lorbeer R, Eur J Heart Fail, 2013).

Growth-Factor-Differentiation-Factor-15 (GDF-15; Swiss Prot Number Q99988) belongs to the PGF beta superfamily and has a role in inflammatory and apoptotic pathways. Serum GDF-15 has been positively correlated with progression of several tumour types, including certain colorectal, pancreatic and prostate cancers. GDF-15 is also upregulated by cardiovascular events triggering oxidative stress, including atherosclerosis. Increasing circulating GDF-15 concentrations have been linked to an enhanced risk of future adverse cardiovascular events.

Chromogranin A (CgA; Swiss Prot Number P10645) is the major soluble protein co-stored and co-released from neurons and neuroendocrine cells together with catecholamines and can function as a pro-hormone by giving rise to several bioactive peptides including vasostatin, pancreastatin, catestatin, parastatin. chromostatin, WE-14 and GE-25. It is also stored in atrial granules in the myocardium. CgA and its fragments exert a broad spectrum of regulatory activities by influencing the endocrine, the cardiovascular, and the immune systems and by affecting the glucose or calcium homeostasis. CgA plasma concentrations associated with all-cause mortality; after adjustment for known risk factors of mortality the association was lost (Rosjo H, Eur J Heart Fail, 2010). CgA is an independent predictor of long-term mortality and heart failure hospitalizations across the spectrum of ACSs and provides incremental prognostic information to conventional cardiovascular risk markers (Jansson AM, Eur Heart J, 2009). CgA can identify subjects with increased risk of short- and long-term mortality (Goetze JP, Endocrine Connections, 2014). Increased CgA (and CT-proET-1) level in subjects with acute destabilized heart failure in the emergency department add independent prognostic information in addition to NT-proBNP measurement (Dieplinger B., Clin Chim Acta, 2009).

Galectin-3 (Swiss Prot Number P17931) is a soluble beta galactosidase binding-lectin secreted by activated macrophages, mast cells and eosinophils, but also by the epithelium of the GI and respiratory tract. It is involved in cell adhesion, chemoattraction, cell growth /differentiation, cell cycle, and apoptosis. Marker of cardiac fibrosis. Association of galectin-3 with new onset heart failure (HF) is stronger in the high risk group (Browers FP, Circ Heart Fail, 2014). Higher concentration of Gal-3 associated with increased risk for incident HF and mortality (Ho JE, JACC, 2012). Galectin-3 is associated with age and risk factors of CV and predicts all-cause mortality in the general population (de Boer RA, J Intern Med, 2011). Elevated levels of plasma galectin-3 associated with increased risks of rapid GFR decline and of incident CKD in the community (O'Seaghdha, J Soc. Nephrol, 2013). Higher levels of galectin-3 are independently associated with all-cause and CVD mortality among community-dwelling older adults with no known CVD at baseline (Daniels LB, Am Heart J, 2014).

Tissue-inhibitors of Metalloproteinases-1 (TIMP-1; Swiss Prot Number P01033) are specific inhibitors that bind matrix metalloproteinases (MMPs). The latter (n = 23) hydrolyze extracellular matrix. There are 4 TIMPs (1-4) whose expression is regulated during development and tissue remodeling. TIMPs (and MMPs) play a central role in many biological processes, such as embryogenesis, normal tissue remodeling, wound healing, and angiogenesis. Higher plasma TIMP-1 (and TIMP-2, -4) level after AMI are associated with MACEs and provide additional prognostic information to that obtained from GRACE clinical risk scores alone (Kelly D, Am J Cardiol, 2010). Strong association between lower levels of circulating TIMP1 (and pro-MMP1) and risk of prevalent CVD, independent from common cardiovascular and inflammatory risk factors (Panayiotou AG, Int Angiol, 2013). TIMP-1 (and MMP-9) correlate with echocardiographic parameters of LV dysfunction and remodelling after AMI and may identify patients at risk of subsequent LV remodelling and adverse prognosis (Kelly D, Eur Heart J, 2008). Circulating concentration of TIMP-1 (and MMP-1) is associated with an extended hypertensive disease, with more target organ damage (Morillas P, J Hypertens, 2013). Apolipoprotein (a) (APOA_HUMAN; Swiss Prot Number P08519) is a part of lipoprotein (a) (Lp (a)). Lipoprotein (a) is a lipoprotein which has a protein component that is composed of apolipoprotein (a) and Apolipoprotein B-100. Apolipoprotein (a) levels are increased in coronary heart diseases (Koch et al. 1997 Journal of the American Society of Nephrology: 1890-1897; Ezhov et al, 2014, Atherosclerosis 235: 477-482).

US2013/0085079 discloses the identification of biomarkers for predicting the risk of future cardiovascular events such as myocardial infarction in a subject. Various markers have been identified, i.a. TFF-3.

In order to be of clinical utility, a new diagnostic marker as a single marker should be comparable to other markers known in the art, or better. It was the object of the present invention to investigate whether a biochemical marker can be identified which may be used in assessing the risk for development of a cardiovascular disease.

Surprisingly, it has been found that the biochemical markers Trefoil Factor 3, alpha-2-Macroglobulin, Vascular Endothelial Growth Factor D (VEGF-D; Swiss Prot Number 043915), Tamm-Horsfall Urine Glycoprotein/Uromoduline (Swiss Prot Number P07911), Kidney-Injury-Molecule-1 (KIM-1; Swiss Prot Number Q96D42), alpha-Glutathione-S-Transferase (Swiss Prot Number P08263), Hepatocyte Growth Factor Receptor (Swiss Prot Number P08581), Neuropilin-1 (Swiss Prot Number 014786) and Insulin-like Growth-Factor Binding Protein 4 (IGFBP4; Swiss Prot Number P22692) are significant predictors alone, in combination with each other or in combination with known biochemical markers for the assessment of the risk of development of a cardiovascular disease within about seven years.

In particular, the inventors have used a randomized pool of patients from the ORIGIN trial and identified in total 8 biochemical markers, i.e. Trefoil Factor 3, alpha-2-Macroglobulin, alpha-Glutathione-S-Transferase, Nt-pro BNP, Peroxiredoxin-4, Osteoprotegerin, Apolipoprotein B and Angiopoietin-2 which alone or in combination with each other significantly correlate with the clinical 1^{st} co-primary endpoint of the ORIGIN study, i.e., cardiovascular death, non-fatal myocardial infarction or non-fatal stroke. These biomarkers have been confirmed when additional factors, such as ACE/ARB or ASA have been included into the basic clinical model.

In order to explore the effect of the basic clinical model on the identity of the above-identified biomarkers of the clinical 1^{st} co-primary endpoint, additional factors, such as HbA1c, Statin or ACE/ARB, ASA and Statin have been included into the basic clinical model, the inventors confirmed Trefoil Factor 3, alpha-2-Macroglobulin and alpha-Glutathione-S-Transferase to significantly correlate with the clinical 1^{st} co-primary endpoint alone or in combination with Nt-pro BNP, Peroxiredoxin-4, Apolipoprotein B, Angiopoietin-2, and Growth-Factor-Differentiation-Factor-15.

In order to explore the effect of the basic clinical model on the identity of the above-identified biomarkers of the clinical 1^{st} co-primary endpoint, additional factors, such as creatinine, have been included into the basic clinical model, the inventors confirmed Trefoil Factor 3 and alpha-Glutathione-S-Transferase to significantly correlate with the clinical 1^{st} co-primary endpoint alone or in combination with Nt-pro BNP, Osteoprotegerin, Angiopoietin-2, Growth-Factor-Differentiation-Factor-15, Chromogranin A and Galectin-3.

In order to explore the effect of the basic clinical model on the identity of the above-identified biomarkers of the clinical 1^{st} co-primary endpoint, additional factors, such as creatinine and HbA1c, have been included into the basic clinical model, the inventors confirmed Trefoil Factor 3 and alpha-Glutathione-S-Transferase to significantly correlate with the clinical 1^{st} co-primary endpoint alone or in combination with Nt-pro BNP, Osteoprotegerin, Angiopoietin-2, Growth-Factor-Differentiation-Factor-15 and Chromogranin A.

For the final validation, the forward selection process was repeated with the full 8401 participants for the 1^{st} co-primary outcome. The inventors confirmed Trefoil Factor 3, alpha-2-Macroglobulin and alpha-Glutathione-S-Transferase and additionally identified Hepatocyte Growth Factor Receptor to significantly correlate with the clinical first co-primary endpoint alone or in combination with Nt-pro-BNP, Angiopoietin-2, Apolipoprotein B, Osteoprotegerin, Growth-Factor-Differentiation-Factor-15, and Chromogranin A.

When age and both age and creatinine were added to the basic clinical model of all participants, the inventors confirmed Trefoil Factor 3, alpha-2-Macroglobulin and alpha-Glutathione-S-Transferase to significantly correlate with the clinical 1^{st} co-primary endpoint alone or in combination with Nt-pro-BNP, Angiopoietin-2, Apolipoprotein B, Growth-Factor-Differentiation-Factor-15, Peroxiredoxin-4 Chromogranin A and Galectin-3.

When creatinine was added to the basic clinical model of all participants and the inventors again identified Trefoil Factor 3, alpha-2-Macroglobulin, alpha-Glutathione-S-Transferase in addition to Neuropilin 1 to significantly correlate with the clinical 1^{st} co-primary endpoint alone or in combination with Nt-pro-BNP, Angiopoietin-2, Apolipoprotein B, Growth-Factor-Differentiation-Factor-15, Osteoprotegerin, Peroxiredoxin-4 Chromogranin A and Galectin-3.

The inventors also identified in total 7 biochemical markers, i.e., Vascular Endothelial Growth Factor D, Tamm-Horsfall Urine Glycoprotein, Kidney-Injury-Molecule-1, alpha-Glutathione-S-Transferase, Growth-Factor-Differentiation-Factor-15 (GDF-15; Swiss Prot Number Q99988), Nt-pro BNP, and Angiopoietin-2 in a randomized pool of patients from the ORIGIN trial which alone or in combination with each other significantly correlate with the clinical 2^{nd} co-primary endpoint of the ORIGIN study, i.e., cardiovascular death, non-fatal myocardial infarction, non-fatal stroke, revascularization procedure or hospitalization for heart failure.

In a preferred embodiment, for the final validation, the forward selection process was repeated with the full 8401 participants for the 2^{nd} co-primary endpoint. The inventors confirmed alpha-Glutathione-S-Transferase and additionally identified Hepatocyte Growth Factor Receptor and Insulin-like Growth Factor Binding Protein 4 to significantly correlate with the second coprimary endpoint alone or in combination with Nt-pro BNP, Angiopoietin-2, Growth-Factor-Differentiation-Factor-15, Apolipoprotein B, Osteoprotegerin and Chromogranin A.

When creatinine was added to the basic clinical model of all participants, the inventors identified alpha-Glutathione-S-Transferase and Hepatocyte Growth Factor Receptor to significantly correlate with the 2^{nd} co-primary endpoint alone or in combination with Nt-pro BNP, Angiopoietin-2, Growth-Factor-Differentiation-Factor-15, Apolipoprotein B, Osteoprotegerin and Chromogranin A.

When age was added to the basic clinical model of all participants and the inventors confirmed Tamm-Horsfall Urine Glycoprotein to significantly correlate with the 2^{nd} co-primary endpoint alone or in combination with Nt-pro BNP, Growth-Factor-Differentiation-Factor-15, Apolipoprotein B, Chromogranin A, Tissue-Inhibitor of Metalloproteinases 1 and Apolipoprotein (a).

When both age and creatinine were added to the basic clinical model of all participants and the inventors confirmed alpha-Glutathione-S-Transferase to significantly correlate with the 2^{nd} coprimary endpoint alone or in combination with Nt-pro BNP, Angiopoietin-2, Growth-Factor-Differentiation-Factor-15, Apolipoprotein B, Chromogranin A and Tissue-Inhibitor of Metalloproteinases-1.

Trefoil Factor 3 (TFF3; Swiss Prot Number Q07654) is a protein that in humans is encoded by the TFF3 gene (chromosome 21). Members of the Trefoil family are characterized by having at least one copy of the Trefoil motif, a 40-AA domain that contains three conserved SS-bonds. Trefoil factors are secretory products of mucin producing cells. They play a key role in the maintenance of the surface integrity of oral mucosa and enhance healing of the gastrointestinal mucosa. TFF comprises the gastric peptides (TFF1), spasmolytic peptide (TFF2), and the intestinal Trefoil factor (TFF3). An involvement of TTF3 in cardiovascular events has not been reported.

Alpha-2-Macroglobulin (Swiss Prot Number P01023) is a large plasma protein found in the blood and is produced by the liver. It acts as an antiprotease and is able to inactivate an enormous variety of proteinases. It is an acute-phase protein. It functions also as a carrier of cytokines, growth factors and hormones. Alpha-2-Macroglobulin rises 10-fold or more in serum in the nephrotic syndrome. The loss of alpha-2-Macroglobulin into urine is prevented by its large size. An involvement of alpha-2-Macroglobulin in cardiovascular events has not been reported. The human alpha class Glutathione-S-Transferases (GSTs) consist of 5 genes, hGSTA1-hGSTA5, and 7 pseudogenes on chromosome 6. Glutathione-S-Transferases (GSTs) comprise a family of eukaryotic and prokaryotic phase II metabolic isozymes best known for their ability to catalyze the conjugation of the reduced form of glutathione (GSH) to xenobiotic substrates for the purpose of detoxification. The mammalian GSTs active in drug metabolism are now classified into the alpha, mu and pi classes. An involvement of alpha-Glutathione-S-Transferase (Swiss Prot Number P08263) in cardiovascular events has not been reported.

Hepatocyte Growth Factor Receptor (HGFR; Swiss Prot Number P08581) is a heterodimeric membrane receptor of mesenchymal origin. Upon stimulation, it has mitogenic, antiapoptotic and angiogenic properties with effects on various cell types. It plays a role in embryonic development and wound healing. Hepatocyte growth factor (HGF) is the only known ligand of HGFR. Subjects with high level of the ligand, HGF, had more CV disease and higher mortality (Kämppä N, Exp Gerontol, 2013). Serum levels of the ligand, HGF, correlate with CHF severity and are associated with CV mortality (Lamblin N, Circulation, 2005). The ligand, HGF, is high in serum of bypass surgery patients with ischemic cardiomyopathy and is a mediator of cardiac stem cells migration (D'Amario, D, Circulation, 2014). High level of HGFR (and its ligand HGF) may reflect attempts to repair failures in organ systems.

Neuropilin-1 (NRP-1; Swiss Prot Number 014786) is one of two human neuropilins expressed by endothelial cells and smooth muscele cells, among other cell types. It is a novel membranebound coreceptor to a tyrosine kinase receptor for both vascular endothelial growth factor (VEGF) and semaphorin. NRP1 plays versatile roles in angiogenesis, axon guidance, cell survival, migration, and invasion. It is essential for blood vessel development in vertebrates. Autologous saphenous vein grafts, unlike internal mammary artery grafts, have lower NRP1 expression and abundant adventitial distribution of NRP1 within their walls; this may correlate with higher susceptibility to intimal hyperplasia (IH) development). IH is the main pathology underlying graft failure following coronary artery bypass graft surgeries for ischaemic heart diseases (Alattar M, Eur J Cardiothorac Surg, 2014). An involvement of Neuropilin-1 in cardiovascular events has not been reported.

Insulin-like Growth Factor Binding Protein 4 (IGFBP-4, Swiss Prot Number P22692) is a member of the insulin-like growth factor binding protein (IGFBP) family (IGFBP-1 to 7) which share 50% homology with each other. IGFBP-4 binds both insulin-like growth factors (IGFs) I and II, proloning the half-life of the IGFs and alters their interaction with cell surface receptors. IGFBP-4 fragments can be utilized as indirect biomarker for MACE prediction in patients with suspected myocardial ischemia (Postnikov AB, Clin Biochem, 2012). Direct detection of full-length IGFBP-4 as biomarker for cardiovascular diseases has not been reported.

Described herein is a method for assessing the risk for development of a cardiovascular disorder in a subject, comprising:
(a) determining in a sample from said subject
   (i) the amount of at least a first marker selected from the group consisting of Trefoil Factor 3, alpha-2-Macroglobulin, Vascular Endothelial Growth Factor D, Tamm-Horsfall Urine Glycoprotein, Kidney-Injury-Molecule-1, Hepatocyte Growth Factor Receptor, Neuropilin-1, Insulin-like Growth Factor Binding Protein 4, and alpha-Glutathione-S-Transferase; and
   (ii) optionally the amount of at least a further marker; and
(b) correlating that said subject is at risk for development of a cardiovascular disorder when said amount is altered compared to a reference amount for the at least first marker.

A "further marker" in the sense of the present invention is any marker that if combined with the first marker adds relevant information in the assessment of the development of a cardiovascular disease. The information is considered relevant if the relative risk of the further marker is similar to the relative risk of the first marker as defined, e.g., by the hazard ratio.

The further marker in the method as described herein may be selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

Further described is a method, e.g., an in vitro method, for assessing the risk, e.g., the increased risk, for development of a cardiovascular disorder in a subject, comprising:
(a) determining in a sample from said subject
   (i) the amount of at least a first marker selected from the group consisting of Trefoil Factor 3, alpha-2-Macroglobulin, and alpha-Glutathione-S-Transferase; and
   (ii) optionally the amount of at least a further marker; and
(b) correlating that said subject is at risk for development of a cardiovascular disorder when said amount is altered compared to a reference amount for the at least first marker.

The further marker in the method as described herein may be selected from the group consisting of Nt-pro BNP, Peroxiredoxin-4, Osteoprotegerin, Apolipoprotein B, and Angiopoietin-2.

The further marker in the method as described herein may be selected from the group consisting of Nt-pro BNP, Peroxiredoxin-4, Apolipoprotein B, Angiopoietin-2, and Growth-Factor-Differentiation-Factor-15.

The further marker in the method as described herein may be selected from the group consisting of Nt-pro BNP, Angiopoietin-2, Apolipoprotein B, Growth-Factor-Differentiation-Factor-15, Peroxiredoxin-4, Chromogranin A and Galectin-3.

Described herein is a method, e.g., an in vitro method, for assessing the risk, e.g., the increased risk, for development of a cardiovascular disorder in a subject, comprising:
(a) determining in a sample from said subject
   (i) the amount of at least a first marker selected from the group consisting of Trefoil Factor 3, and alpha-Glutathione-S-Transferase; and
   (ii) optionally the amount of at least a further marker; and
(b) correlating that said subject is at risk for development of a cardiovascular disorder when said amount is altered compared to a reference amount for the at least first marker.

The further marker in the method as described herein may be selected from the group consisting of Nt-pro BNP, Osteoprotegerin, Angiopoietin-2, Growth-Factor-Differentiation-Factor-15, Chromogranin A and Galectin-3.

The further marker in the method as described herein may be selected from the group consisting of Nt-pro BNP, Osteoprotegerin, Angiopoietin-2, Growth-Factor-Differentiation-Factor-15 and Chromogranin A.

Described herein is a method, e.g., an in vitro method, for assessing the risk, e.g., the increased risk, for development of a cardiovascular disorder in a subject, comprising:
(a) determining in a sample from said subject
   (i) the amount of at least a first marker selected from the group consisting of Trefoil Factor 3, alpha-2-Macroglobulin, alpha-Glutathione-S-Transferase and Hepatocyte Growth Factor Receptor; and
   (ii) optionally the amount of at least a further marker; and
(b) correlating that said subject is at risk for development of a cardiovascular disorder when said amount is altered compared to a reference amount for the at least first marker.

The further marker in the method as described herein may be selected from the group consisting of Nt-pro BNP, Angiopoietin-2, Apolipoprotein B, Osteoprotegerin, Growth-Factor-Differentiation-Factor-15 and Chromogranin A.

Described herein is a method, e.g., an in vitro method, for assessing the risk, e.g., the increased risk, for development of a cardiovascular disorder in a subject, comprising:
(a) determining in a sample from said subject
   (i) the amount of at least a first marker selected from the group consisting of Trefoil Factor 3, alpha-2-Macroglobulin, alpha-Glutathione-S-Transferase and Neuropilin-1; and
   (ii) optionally the amount of at least a further marker; and
(b) correlating that said subject is at risk for development of a cardiovascular disorder when said amount is altered compared to a reference amount for the at least first marker.

The further marker in the method as described herein may be selected from the group consisting of Nt-pro BNP, Angiopoietin-2, Apolipoprotein B, Growth-Factor-Differentiation-Factor-15, Osteoprotegerin, Peroxiredoxin-4, Chromogranin A and Galectin-3.

The present invention relates to a method, e.g., an in vitro method, for assessing the risk, e.g., the increased risk, for development of a cardiovascular disorder in a subject, comprising:
(a) determining in a sample from said subject
   (i) the amount of the first markers alpha-Glutathione-S-Transferase, Insulin-like Growth Factor Binding Protein-4 and Hepatocyte Growth Factor Receptor; and
   (ii) the amount of the further markers Nt-pro BNP, Angiopoietin-2, Growth-Factor-Differentiation-Factor-15, Apolipoprotein B, Osteoprotegerin and Chromogranin A; and
(b) correlating that said subject is at risk for development of a cardiovascular disorder when said amount is altered compared to a reference amount for the first and further markers.

Described herein is a method, e.g., an in vitro method, for assessing the risk, e.g., the increased risk, for development of a cardiovascular disorder in a subject, comprising:
(a) determining in a sample from said subject
   (i) the amount of at least a first marker selected from the group consisting of alpha-Glutathione-S-Transferase and Hepatocyte Growth Factor Receptor; and
   (ii) optionally the amount of at least a further marker; and
(b) correlating that said subject is at risk for development of a cardiovascular disorder when said amount is altered compared to a reference amount for the at least first marker.

The further marker in the method as described herein may be selected from the group consisting of Nt-pro BNP, Angiopoietin-2, Growth-Factor-Differentiation-Factor-15, Apolipoprotein B, Osteoprotegerin and Chromogranin A.

Described herein is a method, e.g., an in vitro method, for assessing the risk, e.g., the increased risk, for development of a cardiovascular disorder in a subject, comprising:
(a) determining in a sample from said subject
   (i) the amount of at least a first marker which is alpha-Glutathione-S-Transferase; and
   (ii) optionally the amount of at least a further marker; and
(b) correlating that said subject is at risk for development of a cardiovascular disorder when said amount is altered compared to a reference amount for the at least first marker.

The further marker in the method as described herein is selected from the group consisting of Nt-pro BNP, Angiopoietin-2, Growth-Factor-Differentiation-Factor-15, Apolipoprotein B, Chromogranin A and Tissue-Inhibitor of Metalloproteinases 1.

Described herein is a method, e.g., an in vitro method, for assessing the risk, e.g., the increased risk, for development of a cardiovascular disorder in a subject, comprising:
(a) determining in a sample from said subject
   (i) the amount of at least a first marker which is Tamm-Horsfall Urine Glycoprotein; and
   (ii) optionally the amount of at least a further marker; and
(b) correlating that said subject is at risk for development of a cardiovascular disorder when said amount is altered compared to a reference amount for the at least first marker.

The further marker in the method as described herein is selected from the group consisting of Nt-pro BNP, Growth-Factor-Differentiation-Factor-15, Apolipoprotein B, Chromogranin A, Tissue-Inhibitor of Metalloproteinases 1 and Apolipoprotein (a).

The first marker may be selected from the group consisting of Trefoil Factor 3, alpha-2-Macroglobulin, Vascular Endothelial Growth Factor D, Tamm-Horsfall Urine Glycoprotein, Kidney-Injury-Molecule-1, Neuropilin-1, Insulin-like Growth Factor Binding Protein 4, and alpha-Glutathione-S-Transferase.

The first marker may be selected from the group consisting of Trefoil Factor 3, alpha-2-Macroglobulin, Vascular Endothelial Growth Factor D, Tamm-Horsfall Urine Glycoprotein, Kidney-Injury-Molecule-1, Neuropilin-1, Hepatocyte Growth Factor Receptor, and alpha-Glutathione-S-Transferase.

The first marker may be selected from the group consisting of Trefoil Factor 3, alpha-2-Macroglobulin, Vascular Endothelial Growth Factor D, Tamm-Horsfall Urine Glycoprotein, Kidney-Injury-Molecule-1, Neuropilin-1, and alpha-Glutathione-S-Transferase.

The first marker may be Trefoil Factor 3, optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be alpha-2-Macroglobulin, optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be alpha-Glutathione-S-Transferase, optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker as described herein may be alpha-Glutathione-S-Transferase and the further markers Nt-pro-BNP and Angiopoietin-2.

The first marker may be Vascular Endothelial Growth Factor D, optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Tamm-Horsfall Urine Glycoprotein, optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Kidney-Injury-Molecule-1, optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Hepatocyte Growth Factor Receptor, optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Insulin-like Growth-Factor Binding Protein 4, optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Neuropilin-1, optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Trefoil Factor 3 and alpha-2-Macroglobulin optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A,

Galectin-3, Tissue-Inhibitor of Metalloproteinases 1 and Angiopoietin-2.

The first marker may be Trefoil Factor 3 and alpha-Glutathione-S-Transferase optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be alpha-2-Macroglobulin and alpha-Glutathione-S-Transferase optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Trefoil Factor 3, alpha-2-Macroglobulin, and alpha-Glutathione-S-Transferase optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Trefoil Factor 3, alpha-2-Macroglobulin, and alpha-Glutathione-S-Transferase optionally with at least a further marker, e.g., wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first and further marker as described herein may be a set of marker comprising Trefoil Factor 3, alpha-2-Macroglobulin, alpha-Glutathione-S-Transferase, Nt-pro BNP, Peroxiredoxin-4, Osteoprotegerin, Apolipoprotein B, and Angiopoietin-2.

The first and further marker as described herein may be a set of marker comprising Trefoil Factor 3, alpha-2-Macroglobulin, alpha-Glutathione-S-Transferase, Nt-pro BNP, Peroxiredoxin-4, Apolipoprotein B, Angiopoietin-2 and Growth-Factor-Differentiation Factor 15.

The first and further marker as described herein may be Trefoil Factor 3, alpha-2-Macroglobulin, alpha-Glutathione-S-Transferase, Nt-pro BNP, Angiopoietin-2, Apolipoprotein B, Growth-Factor-Differentiation-Factor-15, Peroxiredoxin-4, Chromogranin A and Galectin-3.

The first and further marker as described herein may be a set of marker comprising Trefoil Factor 3, alpha-Glutathione-S-Transferase, Nt-pro BNP, Osteoprotegerin, Angiopoietin-2, Growth-Factor-Differentiation-Factor-15, Chromogranin A and Galectin-3,.

The first and further marker as described herein may be Trefoil Factor 3, alpha-Glutathione-S-Transferase, Nt-pro BNP, Osteoprotegerin, Angiopoietin-2, Growth-Factor-Differentiation-Factor-15 and Chromogranin A.

The first marker may be Trefoil-Factor-3 and Hepatocyte-Growth-Factor-Receptor, optionally with at least a further marker, e.g. wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be alpha-2-Macrogobulin and Hepatocyte-Growth-Factor-Receptor, optionally with at least a further marker, e.g. wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be alpha-Glutathione-S-Transferase and Hepatocyte-Growth-Factor-Receptor, optionally with at least a further marker, e.g. wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Trefoil-Factor-3, alpha-2-Macroglobulin and Hepatocyte-Growth-Factor-Receptor, optionally with at least a further marker, e.g. wherein said at least further marker is selected from the group consisting of

Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Trefoil-Factor-3, alpha-Glutathione-S-Transferase and Hepatocyte-Growth-Factor-Receptor, optionally with at least a further marker, e.g. wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be alpha-2-Macroglobulin, alpha-Glutathione-S-Transferase and Hepatocyte-Growth-Factor-Receptor, optionally with at least a further marker, e.g. wherein said at least further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first and further marker as described herein may be a set of marker comprising Trefoil Factor 3, alpha-2-Macroglobulin, alpha-Glutathione-S-Transferase, Hepatocyte Growth Factor Receptor, Nt-pro BNP, Angiopoietin-2, Apolipoprotein B, Osteoprotegerin, Growth-Factor-Differentiation-Factor-15 and Chromogranin A.

The first marker may be Neuropilin-1 and alpha-Glutathione-S-Transferase, optionally with at least a first further marker, e.g. wherein said at least first further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Neuropilin-1 and alpha-2-Macroglobulin, optionally with at least a first further marker, e.g. wherein said at least first further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Neuropilin-1 and Trefoil-Factor-3, optionally with at least a first further marker, e.g. wherein said at least first further marker is selected from the group consisting of Nt-pro BNP, Peroxiredoxin-4, Osteoprotegerin,

Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Neuropilin-1, Trefoil-Factor-3 and alpha-2-Macroglobulin, optionally with at least a first further marker, e.g. wherein said at least first further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Neuropilin-1, Trefoil-Factor-3 and alpha-Glutathione-S-Transferase, optionally with at least a first further marker, e.g. wherein said at least first further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first marker may be Neuropilin-1, alpha-2-Macroglobulin and alpha-Glutathione-S-Transferase, optionally with at least a first further marker, e.g. wherein said at least first further marker is selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

The first and further marker as described herein may be a set of marker comprising Trefoil Factor 3, alpha-2-Macroglobulin, alpha-Glutathione-S-Transferase, Neuropilin-1, Nt-pro BNP, Angiopoietin-2, Apolipoprotein B, Growth-Factor-Differentiation-Factor-15, Osteoprotegerin, Peroxiredoxin-4, Chromogranin A and Galectin-3.

The present invention further relates to a method, e.g., an in vitro method, for assessing the risk, e.g., the increased risk, for development of a cardiovascular disorder in a subject, comprising:
(a) determining in a sample from said subject
   (i) the amount of the first markers Vascular Endothelial Growth Factor D, Tamm-Horsfall Urine Glycoprotein, Kidney-Injury-Molecule-1 and alpha-Glutathione-S-Transferase; and
   (ii) the amount of the further markers Nt-pro BNP, Growth-Factor-Differentiation-Factor-15 and Angiopoietin-2; and
(b) correlating that said subject is at risk for development of a cardiovascular disorder when said amount is altered compared to a reference amount for the first and further markers.

It is described that the first
marker may be Vascular Endothelial Growth Factor D, optionally with at least a further marker, e.g., wherein said at least further marker is selected from the group consisting of Nt-pro BNP, Growth-Factor-Differentiation Factor-15 and Angiopoietin-2.

The first marker may be Tamm-Horsfall Urine Glycoprotein, optionally with at least a further marker, e.g., wherein said at least further marker is selected from the group consisting of Nt-pro BNP, Growth-Factor-Differentiation-Factor-15 and Angiopoietin-2.

The first marker may be alpha-Glutathione-S-Transferase, optionally with at least a further marker, e.g., wherein said at least further marker is selected from the group consisting of Nt-pro BNP, Growth-Factor-Differentiation-Factor-15 and Angiopoietin-2.

The first marker as described herein may be alpha-Glutathione-S-Transferase with the further markers Nt-pro-BNP and Angiopoietin-2.

The first marker may be Kidney-Injury-Molecule-1, optionally with at least a further marker, e.g., wherein said at least further marker is selected from the group consisting of Nt-pro BNP, Growth-Factor-Differentiation-Factor-15 and Angiopoietin-2.

The first marker may be Vascular Endothelial Growth Factor D and Tamm-Horsfall Urine Glycoprotein optionally with at least a further marker, e.g., wherein said at least further marker is selected from the group consisting of Nt-pro BNP, Growth-Factor-Differentiation-Factor-15 and Angiopoietin-2.

The first marker may be Vascular Endothelial Growth Factor D and and alpha-Glutathione-S-Transferase optionally with at least a further marker, e.g., wherein said at least further marker is selected from the group consisting of Nt-pro BNP, Growth-Factor-Differentiation-Factor-15 and Angiopoietin-2.

The first marker may be Vascular Endothelial Growth Factor D and and Kidney-Injury-Molecule-1 optionally with at least a further marker, e.g., wherein said at least further marker is selected from the group consisting of Nt-pro BNP, Growth-Factor-Differentiation-Factor-15 and Angiopoietin-2.

The first marker may be Tamm-Horsfall Urine Glycoprotein and Kidney-Injury-Molecule-1 optionally with at least a further marker, e.g., wherein said at least further marker is selected from the group consisting of Nt-pro BNP, Growth-Factor-Differentiation-Factor-15 and Angiopoietin-2.

The first marker may be Tamm-Horsfall Urine Glycoprotein and alpha-Glutathione-S-Transferase optionally with at least a further marker, e.g., wherein said at least further marker is selected from the group consisting of Nt-pro BNP, Growth-Factor-Differentiation-Factor-15.

The first marker may be Kidney-Injury-Molecule-1 and alpha-Glutathione-S-Transferase optionally with at least a further marker, e.g., wherein said at least further marker is selected from the group consisting of Nt-pro BNP, Growth-Factor-Differentiation-Factor-15 and Angiopoietin-2.

The first marker may be Vascular Endothelial Growth Factor D, Tamm-Horsfall Urine Glycoprotein and Kidney-Injury-Molecule-1 optionally with at least a further marker, e.g., wherein said at least further marker is selected from the group consisting of Nt-pro BNP, Growth-Factor-Differentiation-Factor-15 and Angiopoietin-2.

The first marker may be Vascular Endothelial Growth Factor D, Tamm-Horsfall Urine Glycoprotein and alpha-Glutathione-S-Transferase optionally with at least a further marker, e.g., wherein said at least further marker is selected from the group consisting of Nt-pro BNP, Growth-Factor-Differentiation-Factor-15 and Angiopoietin-2.

The first marker may be Vascular Endothelial Growth Factor D, Kidney-Injury-Molecule-1 and alpha-Glutathione-S-Transferase optionally with at least a further marker, e.g., wherein said at least further marker is selected from the group consisting of Nt-pro BNP, Growth-Factor-Differentiation-Factor-15 and Angiopoietin-2.

The first marker may be Tamm-Horsfall Urine Glycoprotein, Kidney-Injury-Molecule-1 and alpha-Glutathione-S-Transferase optionally with at least a further marker, e.g., wherein said at least further marker is selected from the group consisting of Nt-pro BNP, Growth-Factor-Differentiation-Factor-15 and Angiopoietin-2. In a further preferred embodiment of the invention the first markers are Vascular Endothelial Growth Factor D, Tamm-Horsfall Urine Glycoprotein, Kidney-Injury-Molecule-1 and alpha-Glutathione-S-Transferase with further further markers being Nt-pro BNP, Growth-Factor-Differentiation-Factor-15 and Angiopoietin-2.

In a further preferred embodiment of the invention, the first and further markers as described herein are Vascular Endothelial Growth Factor D, Tamm-Horsfall Urine Glycoprotein, Kidney-Injury-Molecule-1, alpha-Glutathione-S-Transferase, Nt-pro BNP, Growth-Factor-Differentiation-Factor-15 and Angiopoietin-2.

In a further preferred embodiment of the invention, the first and further markers as described herein are alpha-Glutathione-S-Transferase, Insulin-like Growth Factor Binding Protein-4, Hepatocyte Growth Factor Receptor, Nt-pro BNP, Angiopoietin-2, Growth-Factor-Differentiation-Factor-15, Apolipoprotein B, Osteoprotegerin and Chromogranin A.

The first and further markers as described herein may be alpha-Glutathione-S-Transferase, Hepatocyte Growth Factor Receptor, Nt-pro BNP, Angiopoietin-2, Growth-Factor-Differentiation-Factor-15, Apolipoprotein B, Osteoprotegerin and Chromogranin A.

The first and further markers as described herein may be alpha-Glutathione-S-Transferase, Nt-pro BNP, Angiopoietin-2, Growth-Factor-Differentiation-Factor-15, Apolipoprotein B, Chromogranin A and Tissue-Inhibitor of Metalloproteinases 1.

The first and further markers as described herein may be Tamm-Horsfall Urine Glycoprotein, Nt-pro BNP, Growth-Factor-Differentiation-Factor-15, Apolipoprotein B, Chromogranin A, Tissue-Inhibitor of Metalloproteinases 1 and Apolipoprotein (a).

In a preferred embodiment, the method according to the present invention is used for assessing a risk for developing a cardiovascular disease in subjects without a prior diagnosis of a cardiovascular disorder. Alternatively, the subject is known to be at higher than average risk of a cardiovascular disorder, such as a subject fulfilling one or more of the risk factors as defined in a heart study investigating risk factors, e.g. the Interheart Study (Yusuf et al., 2004, Lancet 364:953-962) or the Framingham Heart Study. Such a subject might suffer from one or more of the risk factors selected from the group consisting of a previous diagnosis of a cardiovascular disorder, albuminuria, male, at least 50 years of age, elevated blood cholesterol levels (e.g. LDL levels above 100 mg/dl (2.5 mmol/L)), obesity, preferably abdominal obesity, smoker, diabetic, e.g., type 1, preferably LADA or type 2 elevated HbA1c levels (i.e. > 6.5%), elevated blood, e.g. serum creatinine levels (i.e. >1.0 mg/dl for females and >1.2 mg/dl for males in blood serum) and/or hypertension. In a preferred embodiment such a subject suffers from one or more of the risk factors selected from the group consisting of a previous cardiovascular disorder, albuminuria, male, age of at least 50 years, smoker, diabetic or pre-diabetic, elevated blood cholesterol, elevated blood creatinine, elevated HbA1c levels, obesity and hypertension.

In another preferred embodiment the method according to the present invention is used for assessing a risk for developing a cardiovascular disorder in subjects which had a previous diagnosis of a cardiovascular disorder in the past, are pre-diabetic or diabetic, preferably LADA, or have an age of at least 50 years. A particular preferred embodiment relates to the method according to the present invention that is used for assessing a risk for developing a cardiovascular disorder in subjects which had a previous cardiovascular disorder in the past and are pre-diabetic or diabetic, preferably LADA, and have an age of at least 50 years.

The subject might be a human or non-human animal, such as monkey, rabbit, rat or mouse. The term "pre-diabetic" or "pre-diabetes" as used throughout the application refers, e.g., to a patient with impaired glucose tolerance (IGT), defined as a PPG value ≥140 and <200 mg/dL (ie, ≥7.8 and <11.1 mmol/L), with a FPG <126 mg/dL (7.0 mmol/L) as determined by an oral glucose tolerance test (OGTT) or a patient with impaired fasting glucose (IFG), defined as an FPG ≥110 and <126 mg/dL (≥6.1 and <7 mmol/L), without diabetes mellitus (PPG must be <200 mg/dL [11.1 mmol/L]) both as determined by, e.g., a 75 g oral glucose tolerance test (OGTT) which is known in the art. For example, an (OGTT) is performed fasting (ie, no consumption of food or beverage other than water for at least 8 hours). Two plasma glucose values are drawn during the OGTT - a fasting value (FPG) and a value drawn two hours after the 75 g oral glucose load was administered (postprandial plasma glucose [PPG]). It also refers to a patient with early type 2 diabetes, defined as a FPG ≥126 mg/dL (7.0 mmol/L) or a PPG of ≥200 mg/dL (11.1 mmol/L).

The term "diabetic" as used throughout the application refers to a subject with type 2 diabetes. The term also refers to a subject with type 1 diabetes, preferably with latent autoimmune diabetes (LADA), e.g., as diagnosed by measuring of autoantibodies against, e.g., GAD as described, e.g., in Naik et al., 2009, J Clin Endocrinol Metab, 94:4635-4644.

The term "cardiovascular disorder" as used throughout the application refers to myocardial infarction, stroke, heart failure, revascularization, e.g., of coronary, carotid or peripheral artery, angina pectoris, left ventricular hypertrophy, stenosis, e.g., of coronary, carotid, or lower extremity arteries. Preferably, the cardiovascular disorder develops within the next 2 to 8, 3 to 6 or 5 to 7 years, e.g. from the time point of diagnosis by a method as described herein.

The term "previous cardiovascular disorder" as used throughout the application might refer to a patient with the diagnosis of one or more of the following disorders: myocardial infarction (MI); previous stroke; coronary, carotid or peripheral arterial revascularization; angina with documented ischemic changes (at least 2 mm ST segment depression on electrocardiogram during a Graded Exercise Test [GXT]; or with a cardiac imaging study positive for ischemia); or unstable angina with documented ischemic changes (either ST segment depression of at least 1 mm or an increase in troponin above the normal range but below the range diagnostic for acute myocardial infarction); microalbuminuria or clinical albuminuria (an albumin: creatininee ratio ≥ 30 µg/mg in at least one or timed collection of urine with albumin excretion ≥20 µg/min or ≥30 mg/24 hours or total protein excretion ≥500 mg/24 hours); left ventricular hypertrophy by electrocardiogram or echocardiogram; significant stenosis on angiography of coronary, carotid, or lower extremity arteries (ie, 50% or more stenosis); and/or ankle-brachial index < 0.9.

The method for assessing the risk for the development of a cardiovascular disorder as described herein comprises determining in a sample the amount, e.g. presence, level and/or concentration of at least a first marker as described herein and (ii) optionally the amount of at least a further marker as described herein; and (b) correlating that said subject is at risk for development of a cardiovascular disorder when said amount is altered compared to a reference amount for the at least first marker.

The term "determining" comprises a qualitative determination of the amount of said first or further marker as described herein in a sample compared to a reference amount. In a preferred embodiment the determination is a qualitative or semi-quantitative determination, i.e. it is determined whether the concentration of said first or further marker as described herein is above or below a cut off value. As the skilled artisan will appreciate, in a Yes- (presence) or No-(absence) assay, the assay sensitivity is usually set to match the cut-off value. A cut-off value can for example be determined from the testing of a control population. The control population might be a population of randomized subjects regarding e.g., sex, age, risk factors for developing a cardiovascular disorder as described herein, e.g. smoking, hypertonia, obesity, elevated blood cholesterol levels, pre-diabetes, diabetes and/or increased alcohol consumption. Preferably, the cut-off is set to result in a specificity of 90%, also preferred the cut-off is set to result in a specificity of 95%, or also preferred the cut-off is set to result in a specificity of 98%. Presence of a value above the cut-off value can for example be indicative for the presence of a risk for developing a cardiovascular disorder. Alternatively, it is determined whether the concentration of said first or further marker as described herein is within a specific predefined concentration range of said marker and correlated whether the specific predefined range is associated with a risk for development of a cardiovascular disorder, e.g. by applying unadjusted and adjusted Cox regression models. For example, the marker concentration range which is found within the whole population is predefined in categories, e.g. 3 categories (tertiles), 4 categories (quartiles) or 5 categories (quintiles), preferably quintiles, with category 1 defining the lowest concentration sub-range and category 5 defining the highest concentration sub-range. The risk for development of a cardiovascular disorder, e.g. increases from category 1 to 3-5, respectively.

Alternatively, the term "determining" comprises a quantitative determination of the amount of said first marker as described herein. In this embodiment, the concentration of the marker as described herein is correlated to an underlying diagnostic question like, e.g., stage of disease, disease progression, follow-up after a previous cardiovascular disorder or response to therapy.

As obvious to the skilled artisan, the present invention shall not be construed to be limited to the full-length protein of the first or further marker as described herein. Physiological or artificial fragments of the first or further marker as described herein, secondary modifications of the first or further marker as described herein, as well as allelic variants of the first or further marker as described herein are also encompassed by the present invention. Artificial fragments preferably encompass a peptide produced synthetically or by recombinant techniques, which at least comprises one epitope of diagnostic interest consisting of at least 6 contiguous amino acids as derived from the first or further marker as described herein. Such fragment may advantageously be used for generation of antibodies or as a standard in an immunoassay. More preferred the artificial fragment comprises at least two epitopes of interest appropriate for setting up a sandwich immunoassay.

The full-length proteins as described herein are defined by the sequence according to their data base entry numbers, e.g. SwissProt or Uniprot as described elsewhere herein.

The articles "a" and "an" are used herein to refer to one or to more than one (i.e. to at least one) of the grammatical object of the article. By way of example, "a marker" means one marker or more than one marker. The term "at least" is used to indicate that optionally one or more further objects may be present. By way of example, a marker panel comprising as markers at least the first marker Trefoil Factor 3 and as at least further marker Nt-pro BNP may optionally comprise one or more other markers.

The term "marker" or "biochemical marker" as used herein refers to a molecule to be used as a target for analyzing a patient's test sample. In one embodiment examples of such molecular targets are proteins or polypeptides. In another embodiment such a molecular target can also be non-protein molecules. Proteins or polypeptides used as a marker in the present invention are contemplated to include naturally occurring variants of said protein as well as fragments of said protein or said variant, in particular, immunologically detectable fragments. Immunologically detectable fragments preferably comprise at least 6, 7, 8, 10, 12, 15 or 20 contiguous amino acids of said marker polypeptide. One of skill in the art would recognize that proteins which are released by cells or present in the extracellular matrix may be damaged, e.g., during inflammation, and could become degraded or cleaved into such fragments. Certain markers are synthesized in an inactive form, which may be subsequently activated by proteolysis. As the skilled artisan will appreciate, proteins or fragments thereof may also be present as part of a complex. Such complex also may be used as a marker in the sense of the present invention. Variants of a marker polypeptide are encoded by the same gene, but may differ in their isoelectric point (=PI) or molecular weight (=MW), or both e.g., as a result of alternative mRNA or pre-mRNA processing. The amino acid sequence of a variant is to 80%, 85%, 90%, 95% or more identical to the corresponding marker sequence, particularly the full-length marker sequence of the marker as described herein. In addition, or in the alternative a marker polypeptide or a variant thereof may carry a post-translational modification. Preferred posttranslational modifications are glycosylation, acylation, and/or phosphorylation.

In one embodiment the amount of the marker Insulin-like Growth Factor Binding Protein-4 is the amount that is determined from direct protein expression of Insulin-like Growth Factor Binding Protein-4.

In a further embodiment the amount of the marker Insulin-like Growth Factor Binding Protein-4 that is determined is the full-length Insulin-like Growth Factor Binding Protein-4 as described herein and/or a variant that is to 80%, 85%, 90%, 95% or more identical to the corresponding full-length marker sequence of Insulin-like Growth Factor Binding Protein-4 as described herein.

Preferably, the first or further markers as described herein are specifically measured from a sample by use of a specific binding agent.

A specific binding agent has at least an affinity of 10⁷l/mol for its corresponding target molecule.

The specific binding agent preferably has an affinity of 10⁸ l/mol or also preferred of 10⁹ l/mol for its target molecule. As the skilled artisan will appreciate, the term specific is used to indicate that other biomolecules present in the sample do not significantly bind to the binding agent specific for the marker. Preferably, the level of binding to a biomolecule other than the target molecule results in a binding affinity which is at most only 10% or less, only 5% or less, only 2% or less or only 1% or less of the affinity to the target molecule, respectively. A preferred specific binding agent will fulfil both the above minimum criteria for affinity as well as for specificity.

A specific binding agent preferably is an antibody reactive with the first or further marker as described herein. The term antibody refers to a polyclonal antibody, a monoclonal antibody, antigen binding fragments of such antibodies, single chain antibodies as well as to genetic constructs comprising the binding domain of an antibody.

Any antibody fragment retaining the above criteria of a specific binding agent can be used. Antibodies are generated by state of the art procedures, e.g., as described in Tijssen (Tijssen, P., Practice and theory of enzyme immunoassays, 11, Elsevier Science Publishers B.V., Amsterdam, the whole book, especially pages 43-78). In addition, the skilled artisan is well aware of methods based on immunosorbents that can be used for the specific isolation of antibodies. By these means the quality of polyclonal antibodies and hence their performance in immunoassays can be enhanced. (Tijssen, P., *supra*, pages 108-115).

For the achievements as disclosed in the present invention polyclonal antibodies raised in rabbits may be used. However, clearly also polyclonal antibodies from different species, e.g., rats or guinea pigs, as well as monoclonal antibodies can also be used. Since monoclonal antibodies can be produced in any amount required with constant properties, they represent ideal tools in development of an assay for clinical routine. The generation and the use of monoclonal antibodies to the first or further marker as described herein in a method according to the present invention, respectively, represent yet other preferred embodiments.

As the skilled artisan will appreciate, now that the first or further marker as described herein has been identified as a marker which is useful in the assessment of the risk for the development of a cardiovascular disorder, various immunodiagnostic procedures may be used to reach a result comparable to the achievements of the present invention. For example, alternative strategies to generate antibodies may be used. Such strategies comprise amongst others the use of synthetic peptides, representing an epitope of the first or further marker as described herein for immunization. Alternatively, DNA immunization also known as DNA vaccination may be used.

For determining in the sample obtained from a subject said first or further marker, the sample is incubated with the specific binding agent for said first or further marker under conditions appropriate for formation of a binding agent marker-complex. Such conditions need not be specified, since the skilled artisan without any inventive effort can easily identify such appropriate incubation conditions. The amount of binding agent marker-complex is measured and used in the assessment of the risk for development of a cardiovascular disease. As the skilled artisan will appreciate there are numerous methods to measure the amount of the specific binding agent marker-complex all described in detail in relevant textbooks (cf., e.g., Tijssen P., *supra*, or Diamandis, E. P. and Christopoulos, T. K. (eds.), Immunoassay, Academic Press, Boston (1996)).

For example, the marker as described herein is detected in a sandwich type assay format. In such assay a first specific binding agent is used to capture the marker on the one side and a second specific binding agent, which is labelled to be directly or indirectly detectable, is used on the other side.

In a preferred embodiment, measurement of the marker as described herein in a sample is carried out by using a sandwich immunoassay, wherein Streptavidin-coated microtiter plates are used. A biotinylated polyclonal antibody to marker as described herein is used as a capturing antibody and a digoxigenylated polyclonal antibody to said marker is used as the second specific binding partner in this sandwich assay. The sandwich complex formed is finally visualized by an anti-digoxigenin horseradish peroxidise conjugate and an appropriate peroxidise substrate.

As mentioned above, the first or further marker can be determined from a liquid sample obtained from a subject sample.

In a preferred embodiment the method according to the present invention is practised with blood serum as liquid sample material.

The term "sample" as used herein refers to a biological sample obtained for the purpose of evaluation. In the methods of the present invention, the sample or subject's sample preferably may comprise any body fluid or a tissue extract. For example, test samples include blood, serum, plasma, cerebrospinal fluid and salvia. Preferred samples are whole blood, serum, or plasma, with serum being most preferred.

In one embodiment, the assessment is made in vitro. The subject sample is discarded afterwards. The subject sample is solely used for the in vitro method of the invention and the material of the subject sample is not transferred back into the subject's body. Typically, the sample is a liquid sample, e.g., whole blood, serum, or plasma, preferably serum.

Biochemical markers can either be determined individually or in a preferred embodiment of the invention they can be measured simultaneously using a chip or a bead based array technology. The concentrations of the biomarkers are then either interpreted independently, e.g., using an individual cut-off for each marker or reference amount, or they are combined for interpretation.

The data established indicate that the markers Trefoil Factor 3, alpha-2-Macroglobulin, Vascular Endothelial Growth Factor D, Tamm-Horsfall Urine Glycoprotein, Kidney-Injury-Molecule-1, Hepatocyte Growth Factor Receptor, Neuropilin-1, Insulin-like Growth Factor Binding Protein 4, and alpha-Glutathione-S-Transferase will form an integral part of a marker panel appropriate for diagnostic purposes.

Also described herein is the use of Trefoil Factor 3, alpha-2-Macroglobulin, Vascular Endothelial Growth Factor D, Tamm-Horsfall Urine Glycoprotein, Kidney-Injury-Molecule-1, Hepatocyte Growth Factor Receptor, Neuropilin-1, Insulin-like Growth Factor Binding Protein 4, and alpha-Glutathione-S-Transferase as a marker of a risk marker panel for development of a cardiovascular disorder.

Described herein is the use of at least one marker, e.g. a first marker as described herein, selected from the group consisting of Trefoil Factor 3, alpha-2-Macroglobulin, Vascular Endothelial Growth Factor D, Tamm-Horsfall Urine Glycoprotein, Kidney-Injury-Molecule-1, Hepatocyte Growth Factor Receptor, Neuropilin-1, Insulin-like Growth Factor Binding Protein 4, and alpha-Glutathione-S-Transferase and optionally at least a further marker as described herein in the assessment of the risk for development of a cardiovascular disorder in a subject, wherein determining an altered amount of said marker in a sample from the subject compared to a reference amount for said marker is indicative for said risk.

Described herein is the use of a marker panel comprising Trefoil Factor 3, alpha-2-Macroglobulin, Vascular Endothelial Growth Factor D, Tamm-Horsfall Urine Glycoprotein, Kidney-Injury-Molecule-1, Hepatocyte Growth Factor Receptor, Neuropilin-1, Insulin-like Growth-Factor Binding Protein 4 and/or alpha-Glutathione-S-Transferase and optionally at least a further marker as described herein in the assessment of the risk for development of a cardiovascular disorder in a subject, wherein determining an altered amount of at least Trefoil Factor 3, alpha-2-Macroglobulin, Vascular Endothelial Growth Factor D, Tamm-Horsfall Urine Glycoprotein, Kidney-Injury-Molecule-1, Hepatocyte Growth Factor Receptor, Neuropilin-1, Insulin-like Growth-Factor Binding Protein 4 and/or alpha-Glutathione-S-Transferase in a sample from the subject compared to a reference amount for at least Trefoil Factor 3, alpha-2-Macroglobulin, Vascular Endothelial Growth Factor D, Tamm-Horsfall Urine Glycoprotein, Kidney-Injury-Molecule-1, Hepatocyte Growth Factor Receptor, Neuropilin-1, Insulin-like Growth-Factor Binding Protein 4 and/or alpha-Glutathione-S-Transferase is indicative for said risk.

A further marker as described herein might be selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

Described herein is the use of at least one marker, e.g. a first marker as described herein, selected from the group consisting of Trefoil Factor 3, alpha-2-Macroglobulin, and alpha-Glutathione-S-Transferase and optionally a further marker as described herein in the assessment of the risk for development of a cardiovascular disorder in a subject, wherein determining an altered amount of said marker in a sample from the subject compared to a reference amount for said marker is indicative for said risk.

Also described is the use of a marker panel comprising Trefoil Factor 3, alpha-2-Macroglobulin, and/or alpha-Glutathione-S-Transferase and optionally at least a further marker as described herein and optionally a further marker as described herein in the assessment of the risk for development of a cardiovascular disorder in a subject, wherein determining an altered amount of at least Trefoil Factor 3, alpha-2-Macroglobulin, and/or alpha-Glutathione-S-Transferase in a sample from the subject compared to a reference amount for at least Trefoil Factor 3, alpha-2-Macroglobulin, and/or alpha-Glutathione-S-Transferase is indicative for said risk.

The further marker may be selected from the group consisting of Nt-pro BNP, Peroxiredoxin-4, Osteoprotegerin, Apolipoprotein B and Angiopoietin-2.

The further marker may be selected from the group consisting of Nt-pro BNP, Peroxiredoxin-4, Apolipoprotein B, Angiopoietin-2 and Growth-Factor-Differentiation-Factor-15.

The further marker may be selected from the group consisting of Nt-pro BNP, Angiopoietin-2, Apolipoprotein B, Growth-Factor-Differentiation-Factor-15, Peroxiredoxin-4, Chromogranin A, and Galectin-3.

Also described is the use of at least one marker, e.g a first marker as described herein selected from the group consisting of Trefoil-Factor-3 and alpha-Glutathione-S-Transferase and optionally a further marker as described herein in the assessment of the risk for development of a cardiovascular disorder in a subject wherein determining an altered amount of said marker in a sample from the subject compared to a reference amount for said marker is indicative for said risk.

Also described isthe use of a marker panel comprising Trefoil-Factor-3 and/or alpha-Glutathione-S-Transferase and optionally at least a further marker as described herein in the assessment of the risk for development of a cardiovascular disorder in a subject, wherein determining an altered of at least Trefoil-Factor-3 and/or alpha-Glutathione-S-Transferase in a sample from the subject compared to a reference amount for at least Trefoil-Factor-3 and/or alpha-Glutathione-S-Transferase is indicative for said risk.

The further marker may be selected from the group consisting of Nt-pro BNP, Osteoprotegerin, Angiopoietin-2, Growth-Factor-Differentiation-Factor-15, Chromogranin A and Galectin-3.

The further marker may be selected from the group consisting of Nt-pro BNP, Osteoprotegerin, Angiopoietin-2, Growth-Factor-Differentiation-Factor-15 and Chromogranin A.

Also described is the use of at least one marker, e.g a first marker as described herein selected from the group consisting of Trefoil-Factor-3, alpha-2-Macroglobulin, alpha-Glutathione-S-Transferase, and Hepatocyte Growth Factor Receptor and optionally a further marker as described herein in the assessment of the risk for development of a cardiovascular disorder in a subject wherein determining an altered amount of said marker in a sample from the subject compared to a reference amount for said marker is indicative for said risk.

Also described is the use of a marker panel comprising Trefoil-Factor-3, alpha-2-Macroglobulin, alpha-Glutathione-S-Transferase and/or Hepatocyte Growth Factor Receptor and optionally at least a further marker as described herein in the assessment of the risk for development of a cardiovascular disorder in a subject wherein determining an altered amount of at least Trefoil-Factor-3, alpha-2-Macroglobulin, alpha-Glutathione-S-Transferase and/or Hepatocyte Growth Factor Receptor in a sample from the subject compared to a reference amount for at least Trefoil-Factor-3, alpha-2-Macroglobulin, alpha-Glutathione-S-Transferase and/or Hepatocyte Growth Factor Receptor is indicative for said risk.

The further marker may be selected from the group consisting of Nt-pro BNP, Angiopoietin-2, Apolipoprotein B, Osteoprotegerin, Growth-Factor-Differentiation-Factor 15 and Chromogranin A.

Also described is the use of at least one marker, e.g. a first marker as described herein selected from the group consisting of Trefoil-Factor-3, alpha-2-Macroglobulin, alpha-Glutathione-S-Transferase and Neuropilin-1 and optionally a further marker as described herein in the assessment of the risk for development of a cardiovascular disorder in a subject, wherein determining an altered amount of said marker in a sample from the subject compared to a reference amount for said marker is indicative for said risk.

Also described is the use of a marker panel comprising Trefoil Factor 3, alpha-2-Macroglobulin, alpha-Glutathione-S-Transferase and/or Neuropilin-1 and optionally at least one further marker in the assessment of the risk for development of a cardiovascular disorder in a subject, wherein determining an altered amount of at least Trefoil Factor 3, alpha-2-Macroglobulin, alpha-Glutathione-S-Transferase and/or Neuropilin-1 in a sample from the subject compared to a reference amount for at least Trefoil Factor 3, alpha-2-Macroglobulin, alpha-Glutathione-S-Transferase and/or Neuropilin-1 is indicative for said risk.

In another embodiment, the further marker may be selected from the group consisting of Nt-pro BNP, Angiopoietin-2, Apolipoprotein B, Growth-Factor-Differentiation-Factor-15, Osteoprotegerin, Peroxiredoxin-4, Chromogranin A and Galectin-3.

The data established herein also indicate that the markers Vascular Endothelial Growth Factor D, Tamm-Horsfall Urine Glycoprotein, Kidney-Injury-Molecule-1 and alpha-Glutathione-S-Transferase will form an integral part of a marker panel appropriate for diagnostic purposes.

The present invention therefore relates to the use of Vascular Endothelial Growth Factor D, Tamm-Horsfall Urine Glycoprotein, Kidney-Injury-Molecule-1 alpha-Glutathione-S-Transferase, Nt-pro BNP, Growth-Factor Differentiation Factor-15 and Angiopoietin-2 as a marker of a risk marker panel for development of a cardiovascular disorder.

Described herein is the use of markers, e.g. first markers as described herein, from the group consisting of Vascular Endothelial Growth Factor D, Tamm-Horsfall Urine Glycoprotein, Kidney-Injury-Molecule-1 and/or alpha-Glutathione-S-Transferase and further markers from the group consisting of Nt-pro BNP, Growth-Factor-Differentiation-Factor-15 and Angiopoietin-2 in the assessment of the risk for development of a cardiovascular disorder in a subject, wherein determining an altered amount of said markers in a sample from the subject compared to a reference amount for said markers is indicative for said risk.

Described herein is also the use of a marker panel comprising Vascular Endothelial Growth Factor D, Tamm-Horsfall Urine Glycoprotein, Kidney-Injury-Molecule-1 and alpha-Glutathione-S-Transferase and further markers as described herein in the assessment of the risk for development of a cardiovascular disorder in a subject, wherein determining an altered amount of Vascular Endothelial Growth Factor D, Tamm-Horsfall Urine Glycoprotein, Kidney-Injury-Molecule-1 and alpha-Glutathione-S-Transferase in a sample from the subject compared to a reference amount of Vascular Endothelial Growth Factor D, Tamm-Horsfall Urine Glycoprotein, Kidney-Injury-Molecule-1 and alpha-Glutathione-S-Transferase is indicative for said risk. Preferred markers are from the group consisting of Nt-pro BNP, Growth-Factor-Differentiation-Factor-15 and Angiopoietin-2.

Described herein is also the use of at least one marker selected from the group consisting of alpha-Glutathione-S-Transferase, Insulin-like Growth Factor Binding Protein-4 and Hepatocyte Growth Factor Receptor and optionally at least one further marker in the assessment of the risk for development of a cardiovascular disorder in a subject, wherein determining an altered amount of said marker in a sample from the subject compared to a reference amount for said marker is indicative for said risk.

Described herein is also the use of a marker panel comprising alpha-Glutathione-S-Transferase, Insulin-like Growth Factor Binding Protein 4 and Hepatocyte Growth Factor Receptor and further markers as described herein in the assessment of the risk for development of a cardiovascular disorder in a subject wherein determining an altered amount of alpha-Glutathione-S-Transferase, Insulin-like Growth Factor Binding Protein 4 and Hepatocyte Growth Factor Receptor in a sample from the subject compared to a reference amount of alpha-Glutathione-S-Transferase, Insulin-like Growth Factor Binding Protein 4 and Hepatocyte Growth Factor Receptor is indicative for said risk.

Further markers are from the group consisting of Nt-pro BNP, Angiopoietin-2, Growth-Factor-Differentiation-Factor-15, Apolipoprotein B, Osteoprotegerin and Chromogranin A.

Also described is the use of at least one marker selected from the group consisting of alpha-Glutathione-S-Transferase and Hepatocyte Growth Factor Receptor and optionally at least one further marker in the assessment of the risk for development of a cardiovascular disorder in a subject, wherein determining an altered amount of said marker in a sample from the subject compared to a reference amount for said marker is indicative for said risk.

Also described is the use of a marker panel comprising alpha-Glutathione-S-Transferase and/or Hepatocyte Growth Factor Receptor and optionally at least one further marker in the assessment of the risk for development of a cardiovascular disorder in a subject, wherein determining an altered amount of at least alpha-Glutathione-S-Transferase and/or Hepatocyte Growth Factor Receptor in a sample from the subject compared to a reference amount for at least alpha-Glutathione-S-Transferase and/or Hepatocyte Growth Factor Receptor is indicative for said risk.

The further marker may be selected from the group consisting of Nt-pro BNP, Angiopoietin-2, Growth-Factor-Differentiation-Factor-15, Apolipoprotein B, Osteoprotegerin and Chromogranin A.

Also described is the use of at least one marker is alpha-Glutathione-S-Transferase and optionally at least one further marker in the assessment of the risk for development of a cardiovascular disorder in a subject, wherein determining an altered amount of said marker in a sample from the subject compared to a reference amount for said marker is indicative for said risk.

Also described is the use of a marker panel comprising alpha-Glutathione-S-Transferase and optionally at least one further marker in the assessment of the risk for development of a cardiovascular disorder in a subject, wherein determining an altered amount of at least alpha-Glutathione-S-Transferase in a sample from the subject compared to a reference amount for at least alpha-Glutathione-S-Transferase is indicative for said risk.

The further marker may be selected from the group consisting of Nt-pro BNP, Angiopoietin-2, Growth-Factor-Differentiation-Factor-15, Apolipoprotein B, Chromogranin A and Tissue-Inhibitor of Metalloproteinases 1.

Also described is the use of at least one marker is Tamm-Horsfall Urine Glycoprotein and optionally at least one further marker in the assessment of the risk for development of a cardiovascular disorder in a subject, wherein determining an altered amount of said marker in a sample from the subject compared to a reference amount for said marker is indicative for said risk.

Also described is the use of a marker panel comprising Tamm-Horsfall Urine Glycoprotein and optionally at least one further marker in the assessment of the risk for development of a cardiovascular disorder in a subject, wherein determining an altered amount of at least Tamm-Horsfall Urine Glycoprotein in a sample from the subject compared to a reference amount for at least Tamm-Horsfall Urine Glycoprotein is indicative for said risk.

The further marker may be selected from the group consisting of Nt-pro BNP, Growth-Factor-Differentiation-Factor-15, Apolipoprotein B, Chromogranin A, Tissue-Inhibitor of Metalloproteinases 1 and Apolipoprotein (a).

In the uses as described herein a first marker is selected from the group consisting of Trefoil Factor 3, alpha-2-Macroglobulin, Vascular Endothelial Growth Factor D, Tamm-Horsfall Urine Glycoprotein, Kidney-Injury-Molecule-1, Neuropilin-1, Insulin-like Growth Factor Binding Protein 4, and alpha-Glutathione-S-Transferase optionally with at least one further marker as described herein, e.g., selected from the group consisting of Nt-pro BNP, Peroxiredoxin-4, Osteoprotegerin, Apolipoprotein B, Growth-Factor-Differentiation-Factor-15, Chromogranin A, Galectin-3, Tissue-Inhibitor of Metalloproteinases 1 and Angiopoietin-2.

In the uses as described herein a first marker is selected from the group consisting of Trefoil Factor 3, alpha-2-Macroglobulin, Vascular Endothelial Growth Factor D, Tamm-Horsfall Urine Glycoprotein, Kidney-Injury-Molecule-1, Neuropilin-1, Hepatocyte Growth Factor Receptor, and alpha-Glutathione-S-Transferase optionally with at least one further marker as described herein, e.g., selected from the group consisting of Nt-pro BNP, Peroxiredoxin-4, Osteoprotegerin, Apolipoprotein B, Growth-Factor-Differentiation-Factor-15, Chromogranin A, Galectin-3, Tissue-Inhibitor of Metalloproteinases 1 and Angiopoietin-2.

In the uses as described herein a first marker is selected from the group consisting of Trefoil Factor 3, alpha-2-Macroglobulin, Vascular Endothelial Growth Factor D, Tamm-Horsfall Urine Glycoprotein, Kidney-Injury-Molecule-1, Neuropilin-1, and alpha-Glutathione-S-Transferase optionally with at least one further marker as described herein, e.g., selected from the group consisting of Nt-pro BNP, Peroxiredoxin-4, Osteoprotegerin, Apolipoprotein B, Growth-Factor-Differentiation-Factor-15, Chromogranin A, Galectin-3, Tissue-Inhibitor of Metalloproteinases 1 and Angiopoietin-2.

In the uses as described herein a first marker is Trefoil Factor 3, optionally with at least one further marker as described herein, e.g., selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

In the uses as described herein a first marker is alpha-2-Macroglobulin, optionally with at least one further marker as described herein, e.g., selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

In the uses as described herein a first marker is alpha-Glutathione-S-Transferase, optionally with at least one further marker as described herein, , e.g., selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

In the uses as described herein a first marker is Vascular Endothelial Growth Factor D, optionally with at least one further marker as described herein, e.g., selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

In the uses as described herein a first marker is Tamm-Horsfall Urine Glycoprotein, optionally with at least one further marker as described herein, e.g., selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

In the uses as described herein a first marker is Kidney-Injury-Molecule-1, optionally with at least one further marker as described herein, e.g., selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

In the uses as described herein a first marker is Hepatocyte Growth Factor Receptor, optionally with at least one further marker as described herein, e.g., selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

In the uses as described herein a first marker is Insulin-like Growth Factor Binding Protein 4, optionally with at least one further marker as described herein, e.g., selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

In the uses as described herein a first marker is Neuropilin-1, optionally with at least one further marker as described herein, e.g., selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

A use or use of a marker panel may comprise as marker alpha-Glutathione-S-Transferase, Nt-pro BNP and Angiopoietin-2.

A use or use of a marker panel may comprise as marker Trefoil Factor 3, alpha-2-Macroglobulin and optionally at least a further marker. Preferred further markers are selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

A use or use of a marker panel may comprise as marker alpha-2-Macroglobulin, alpha-Glutathione-S-Transferase and optionally at least a further marker. Preferred further markers are selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

A use or use of a marker panel may comprise as marker Trefoil Factor 3, alpha-Glutathione-S-Transferase and optionally at least a further marker. Preferred further markers are selected from the group consisting of Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

A use or use of a marker panel may comprise as marker Trefoil Factor 3, alpha-2-Macroglobulin, alpha-Glutathione-S-Transferase, Nt-pro BNP, Peroxiredoxin-4, Osteoprotegerin, Apolipoprotein B, and Angiopoietin-2.

A use or use of a marker panel may comprise as marker Trefoil Factor 3, alpha-2-Macroglobulin, alpha-Glutathione-S-Transferase, Nt-pro BNP,

Peroxiredoxin-4, Apolipoprotein B, Angiopoietin-2, and Growth-Factor-Differentiation-Factor 15.

A use or use of a marker panel may comprise as marker Trefoil Factor 3, alpha-2-Macroglobulin, alpha-Glutathione-S-Transferase, Nt-pro BNP, Angiopoietin-2, Apolipoprotein B, Growth-Factor-Differentiation-Factor-15, Peroxiredoxin 4, Chromogranin A and Galectin-3.

Another use or use of a marker panel may comprise as marker Trefoil Factor 3, alpha-Glutathione-S-Transferase, Nt-pro BNP, Osteoprotegerin, Angiopoietin-2, Growth-Factor-Differentiation-Factor-15, Chromogranin A and Galectin-3.

Another use or use of a marker panel may comprise as marker Trefoil Factor 3, alpha-Glutathione-S-Transferase, Nt-pro BNP, Osteoprotegerin, Angiopoietin-2, Growth-Factor-Differentiation-Factor-15 and Chromogranin A

Another use or use of a marker panel may comprise as marker Trefoil Factor 3, alpha-2-Macroglobulin, alpha-Glutathione-S-Transferase, Hepatocyte Growth Factor Receptor, Nt-pro BNP, Angiopoietin-2, Apolipoprotein B, Osteoprotegerin, Growth-Factor-Differentiation-Factor-15 and Chromogranin A.

Another use or use of a marker panel may comprise as marker Trefoil Factor 3, alpha-2-Macroglobulin, alpha-Glutathione-S-Transferase, Neuropilin-1, Nt-pro BNP, Angiopoietin-2, Apolipoprotein B, Growth-Factor-Differentiation-Factor-15, Osteoprotegerin, Peroxiredoxin-4, Chromogranin A and Galectin-3.

A marker panel may comprise Vascular Endothelial Growth Factor D and at least a further marker. Further markers are selected from the group consisting of Nt-pro BNP, Growth-Factor-Differentiation-Factor-15 and Angiopoietin-2.

A marker panel may comprise alpha-Glutathione-S-Transferase and at least a further marker. Further markers are selected from the group consisting of Nt-pro BNP, Growth-Factor-Differentiation-Factor-15 and Angiopoietin-2.

A marker panel maycomprise Tamm-Horsfall Urine Glycoprotein and at least a further marker. Further markers are selected from the group consisting of Nt-pro BNP, Growth-Factor-Differentiation-Factor-15 and Angiopoietin-2.

A marker panel comprise Kidney-Injury-Molecule-1 and at least a further marker. Further markers are selected from the group consisting of Nt-pro BNP, Growth-Factor-Differentiation-Factor-15 and Angiopoietin-2.

The marker panel may comprise Vascular Endothelial Growth Factor D and Tamm-Horsfall Urine Glycoprotein and optionally at least a further marker. Further markers are selected from the group consisting of Growth-Factor-Differentiation-Factor-15, Nt-pro BNP, and Angiopoietin-2.

The marker panel may comprise Vascular Endothelial Growth Factor D and alpha-Glutathione-S-Transferase and optionally at least a further marker. Further markers are selected from the group consisting of Growth-Factor-Differentiation-Factor-15, Nt-pro BNP, and Angiopoietin-2.

The marker panel may comprise Vascular Endothelial Growth Factor D and Kidney-Injury-Molecule-1 and optionally at least a further marker. Further markers are selected from the group consisting of Growth-Factor-Differentiation-Factor-15, Nt-pro BNP, and Angiopoietin-2.

The marker panel may comprise Tamm-Horsfall Urine Glycoprotein and Kidney-Injury-Molecule-1 and optionally at least a further marker. Further markers are selected from the group consisting of Growth-Factor-Differentiation-Factor-15, Nt-pro BNP, and Angiopoietin-2.

The marker panel may comprise Tamm-Horsfall Urine Glycoprotein and alpha-Glutathione-S-Transferase and optionally at least a further marker. Further markers are selected from the group consisting of Growth-Factor-Differentiation-Factor-15, Nt-pro BNP, and Angiopoietin-2.

The marker panel may comprise Kidney-Injury-Molecule-1 and alpha-Glutathione-S-Transferase and optionally at least a further marker. Further markers are selected from the group consisting of Growth-Factor-Differentiation-Factor-15, Nt-pro BNP, and Angiopoietin-2.

The marker panel may comprise Vascular Endothelial Growth Factor D, Tamm-Horsfall Urine Glycoprotein and Kidney-Injury-Molecule-1 and optionally at least a further marker. Further markers are selected from the group consisting of Growth-Factor-Differentiation-Factor-15, Nt-pro BNP, and Angiopoietin-2.

The marker panel may comprise Vascular Endothelial Growth Factor D, Tamm-Horsfall Urine Glycoprotein and alpha-Glutathione-S-Transferase and optionally at least a further marker. Further markers are selected from the group consisting of Growth-Factor-Differentiation-Factor-15, Nt-pro BNP, and Angiopoietin-2.

The marker panel may comprise Vascular Endothelial Growth Factor D, Kidney-Injury-Molecule-1 and alpha-Glutathione-S-Transferase and optionally at least a further marker. Further markers are selected from the group consisting of Growth-Factor-Differentiation-Factor-15, Nt-pro BNP, and Angiopoietin-2.

The marker panel may comprise Tamm-Horsfall Urine Glycoprotein, Kidney-Injury-Molecule-1 and alpha-Glutathione-S-Transferase and optionally at least a further marker. Further markers are selected from the group consisting of Growth-Factor-Differentiation-Factor-15, Nt-pro BNP, and Angiopoietin-2.

In a preferred embodiment of the invention, the marker panel comprises Vascular Endothelial Growth Factor D, Tamm-Horsfall Urine Glycoprotein, Kidney-Injury-Molecule-1 and alpha-Glutathione-S-Transferase and further markers from the group consisting of Growth-Factor-Differentiation-Factor-15, Nt-pro BNP, and Angiopoietin-2.

A preferred marker panel of the invention also comprises Vascular Endothelial Growth Factor D, Tamm-Horsfall Urine Glycoprotein, Kidney-Injury-Molecule-1, alpha-Glutathione-S-Transferase, Nt-pro BNP, Growth-Factor-Differentiation-Factor-15 and Angiopoietin-2.

Also described is the use of a marker panel of the invention comprises as marker alpha-Glutathione-S-Transferase, Insulin-like Growth Factor Binding Protein-4, Hepatocyte Growth Factor Receptor, Nt-pro BNP, Angiopoietin-2, Growth-Factor-Differentiation-Factor-15, Apolipoprotein B, Osteoprotegerin and Chromogranin A.

Another use or use of a marker panel may comprise as marker Tamm-Horsfall Urine Glycoprotein, Chromogranin A, Tissue Inhibitor of Metalloproteinases-1, Apolipoprotein (a), Nt-pro BNP, Growth-Factor-Differentiation-Factor-15 and Apolipoprotein B.

Another use or use of a marker panel may comprise as marker alpha-Glutathione-S-Transferase and Hepatocyte Growth Factor Receptor, Nt-pro BNP,
Angiopoietin-2, Growth-Factor-Differentiation-Factor-15, Apolipoprotein B, Osteoprotegerin and Chromogranin A.

Another use or use of a marker panel may comprise as marker alpha-Glutathione-S-Transferase, Nt-pro BNP, Angiopoietin-2, Growth-Factor-Differentiation-Factor-15, Apolipoprotein B, Chromogranin A and Tissue-Inhibitor of Metalloproteinases 1.

As the skilled artisan will appreciate, there are many ways to use the determination of the amount of two or more markers in order to correlate the diagnostic question under investigation. In a quite simple, but nonetheless often effective approach, a positive result, i.e. presence of a risk for the development of a cardiovascular disorders is present, is assumed if in a sample the marker is altered compared to a reference amount. For example, for the markers Trefoil Factor 3, alpha-2-Macroglobulin, Growth-Factor-Differentiation-Factor-15, Vascular Endothelial Growth Factor D, Kidney-Injury-Molecule-1, Nt-pro BNP, Peroxiredoxin-4, Osteoprotegerin, Apolipoprotein B, Insulin-like Growth Factor Binding Protein 4, Tissue Inhibitor of Metalloproteinases 1, Apolipoprotein (a) and Angiopoietin-2 a level above the reference amount predicts a higher risk for the development of a cardiovascular disorder. In contrast, for example, for the markers Glutathione-S-Transferase, Chromogranin A, Hepatocyte Growth Factor Receptor, Galectin-3, Neuropilin-1 and Tamm-Horsfall Urine Glycoprotein a level above the reference amount predicts a lower risk for the development of a cardiovascular disorder.

Frequently, however, the combination of markers is evaluated. Preferably the values measured for markers of a marker panel, e.g. for Trefoil Factor 3 and Nt-pro BNP, are mathematically combined and the combined value is correlated to the underlying diagnostic question. Marker values may be combined by any appropriate state of the art mathematical method. Well-known mathematical methods for correlating a marker combination to a disease employ methods like, discriminant analysis (DA) (i.e. linear-, quadratic-, regularized-DA), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting/Bagging Methods), Generalized Linear Models (i.e. Logistic Regression), Principal Components based Methods (i.e. SIMCA), Generalized Additive Models, Fuzzy Logic based Methods, Neural Networks and Genetic Algorithms based Methods. The skilled artisan will have no problem in selecting an appropriate method to evaluate a marker combination of the present invention. Details relating to these statistical methods are found in the following references: Ruczinski, I., et al, J. of Computational and Graphical Statistics, 12 (2003) 475-511; Friedman, J. H., J. of the American Statistical Association 84 (1989) 165-175; Hastie, Trevor, Tibshirani, Robert, Friedman, Jerome, The Elements of Statistical Learning, Springer Series in Statistics, 2001; Breiman, L., Friedman, J. H., Olshen, R. A., Stone, C. J. (1984) Classification and regression trees, California: Wadsworth; Breiman, L., Random Forests, Machine Learning, 45 (2001) 5-32; Pepe, M. S., The Statistical Evaluation of Medical Tests for Classification and Prediction, Oxford Statistical Science Series, 28 (2003); and Duda, R. O., Hart, P. E., Stork, D. G., Pattern Classification, Wiley Interscience, 2nd Edition (2001).

It is a further disclosed to use an optimized multivariate cut-off for the underlying combination of biological markers and to discriminate state A from state B, e.g. risk from non-risk. In this type of analysis the markers are no longer independent but form a marker panel.

Accuracy of a diagnostic method is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision threshold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results)/(number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/1-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One preferred way to quantify the diagnostic accuracy of a laboratory test is to express its performance by a single number. Such an overall parameter, e.g., is the so-called "total error" or alternatively the "area under the curve = AUC". The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Disclosed are systems and methods for developing diagnostic tests like, for example, a kit (e.g., detection, screening, monitoring, predictive and prognostic tests).

Described is a kit comprising a reagent required to specifically determine at least Trefoil Factor 3, alpha-2-Macroglobulin, Vascular Endothelial Growth Factor D, Tamm-Horsfall Urine Glycoprotein, Kidney-Injury-Molecule-1, Hepatocyte Growth Factor Receptor, Neuropilin-1, Insulin-like Growth Factor Binding Protein 4, and alpha-Glutathione-S-Transferase, optionally at least a further marker and auxiliary reagents for performing the determination.

Described is a kit comprising a reagent required to specifically determine at least the further marker Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2.

Described is a kit comprising a reagent required to specifically determine at least Trefoil Factor 3, alpha-2-Macroglobulin, Vascular Endothelial Growth Factor D, Tamm-Horsfall Urine Glycoprotein, Kidney-Injury-Molecule-1, Neuropilin-1, Insulin-like Growth Factor Binding Protein 4, and alpha-Glutathione-S-Transferase.

Described is a kit comprising a reagent required to specifically determine at least Trefoil Factor 3, alpha-2-Macroglobulin,
Vascular Endothelial Growth Factor D, Tamm-Horsfall Urine Glycoprotein, Kidney-Injury-Molecule-1, Neuropilin-1, Hepatocyte Growth Factor Receptor, and alpha-Glutathione-S-Transferase.

Described is a kit comprising a reagent required to specifically determine at least Trefoil Factor 3, alpha-2-Macroglobulin, Vascular Endothelial Growth Factor D, Tamm-Horsfall Urine Glycoprotein, Kidney-Injury-Molecule-1, Neuropilin-1, and alpha-Glutathione-S-Transferase.

Described is a kit comprising a reagent required to specifically determine at least Trefoil Factor 3, alpha-2-Macroglobulin, alpha-Glutathione-S-Transferase, and optionally auxiliary reagents for performing the determination.

Described is a kit comprising a reagent required to specifically determine at least the further marker Nt-pro BNP, Peroxiredoxin 4, Osteoprotegerin, Apolipoprotein B, and/or Angiopoietin-2, and optionally auxiliary reagents for performing the determination.

Described is a kit comprising a reagent required to specifically determine at least the further marker Nt-pro BNP, Peroxiredoxin 4, Apolipoprotein B, Angiopoietin-2 and/or Growth-Factor-Differentiation-Factor-15, and optionally auxiliary reagents for performing the determination.

Described is a kit comprising a reagent required to specifically determine the further marker Nt-pro BNP, Angiopoietin-2, Apolipoprotein B, Growth-Factor-Differentiation-Factor-15, Peroxiredoxin-4, Chromogranin A and/or Galectin-3.

Described is a kit comprising a reagent required to specifically determine at least Trefoil Factor 3, optionally at least a further marker as described herein, e.g. Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2. and optionally auxiliary reagents for performing the determination.

Described is a kit comprising a reagent required to specifically determine at least alpha-2-Macroglobulin optionally at least a further marker as described herein, e.g. Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2. and optionally auxiliary reagents for performing the determination.

Described is a kit comprising a reagent required to specifically determine at least alpha-Glutathione-S-Transferase optionally at least a further marker as described herein, e.g. Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2. and optionally auxiliary reagents for performing the determination.

Described is a kit comprising a reagent required to specifically determine alpha-Glutathione-S-Transferase, Nt-pro BNP and Angiopoietin-2.

Described is a kit comprising a reagent required to specifically determine at least Trefoil Factor 3, alpha-2-Macroglobulin, alpha-Glutathione-S-Transferase, Nt-pro BNP, Peroxiredoxin 4, Osteoprotegerin, Apolipoprotein B and Angiopoietin-2, and optionally auxiliary reagents for performing the determination.

Described isa kit comprising a reagent required to specifically determine at least Trefoil Factor 3, alpha-2-Macroglobulin, alpha-Glutathione-S-Transferase, Nt-pro BNP, Peroxiredoxin 4, Apolipoprotein B, Angiopoietin-2 and Growth-Factor-Differentiation-Factor-15.

Described is a kit comprising a reagent required to specifically determine at least Trefoil Factor 3, alpha-2- Macroglobulin, alpha-Glutathione-S-Transferase, Chromogranin A, Galectin-3, Nt-pro BNP, Angiopoietin-2, Apolipoprotein B, Growth-Factor-Differentiation-Factor-15, and Peroxiredoxin 4.

Described is a kit comprising a reagent required to specifically determine at least Trefoil Factor 3 and alpha-Glutathione-S-Transferase and optionally at least a further marker and auxiliary reagents for performing the determination.

Further markers may be selected from the group consisting of Nt-pro BNP,
Osteoprotegerin, Angiopoietin-2, Growth-Factor-Differentiation-Factor-15, Chromogranin A and Galectin-3.

Further markers may be selected from the group consisting of Nt-pro BNP, Osteoprotegerin, Angiopoietin-2, Growth-Factor-Differentiation-Factor-15 and Chromogranin A.

Described is a kit comprising a reagent required to specifically determine at least Trefoil Factor 3, Glutathione-S-Transferase, Nt-pro BNP, Osteoprotegerin, Angiopoietin-2, Growth-Factor-Differentiation-Factor-15, Chromogranin A and Galectin-3.

Described is a kit comprising a reagent required to specifically determine at least Trefoil Factor 3, alpha-Glutathione-S-Transferase, Nt-pro BNP, Osteoprotegerin, Angiopoietin-2, Growth-Factor-Differentiation-Factor-15, and Chromogranin A.

Described isa kit comprising a reagent required to specifically determine at least Trefoil Factor 3, alpha-2-Macroglobulin, alpha-Glutathione-S-Transferase and Hepatocyte Growth Factor Receptor, optionally at least a further marker and auxiliary reagents for performing the determination.

Further markers may be selected from the group consisting of Nt-pro BNP, Angiopoietin-2 Apolipoprotein B, Osteoprotegerin, Growth-Factor-Differentiation-Factor-15 and Chromogranin A.

Described is a kit comprising a reagent required to specifically determine at least Hepatocyte Growth Factor Receptor optionally at least a further marker as described herein, e.g. Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2 and optionally auxiliary reagents for performing the determination.

Described is a kit comprising a reagent required to specifically determine at least Trefoil Factor 3, alpha-2-Macroglobulin, alpha-Glutathione-S-Transferase, Chromogranin A, Hepatocyte Growth Factor Receptor, Nt-pro BNP, Angiopoietin-2, Apolipoprotein B, Osteoprotegerin, Growth-Factor-Differentiation-Factor-15.

Described is a kit comprising a reagent required to specifically determine at least Trefoil Factor 3, alpha-2-Macroglobulin, alpha-Glutathione-S-Transferase and/or Neuropilin-1 optionally at least a further marker and auxiliary reagents for performing the determination.

Further markers may be selected from the group consisting of Nt-pro BNP, Angiopoietin-2 Apolipoprotein B, Growth-Factor-Differentiation-Factor-15, Osteoprotegerin, Peroxiredoxin-4, Chromogranin A and Galectin-3.

Described is a kit comprising a reagent required to specifically determine Neuropilin-1 optionally at least a further marker as described herein, e.g. Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2 and optionally auxiliary reagents for performing the determination.

Described is a kit comprising a reagent required to specifically determine at least Trefoil Factor 3, alpha-2- Macroglobulin, alpha-Glutathione-S-Transferase, Chromogranin A, Galectin-3, Neuropilin-1, Nt-pro BNP, Angiopoietin-2, Apolipoprotein B, Growth-Factor-Differentiation-Factor-15, Osteoprotegerin, and Peroxiredoxin 4.

Described is a kit comprising a reagent required to specifically determine at least Vascular Endothelial Growth Factor D, optionally at least a further marker as described herein, e.g. Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2 and optionally auxiliary reagents for performing the determination.

Described is a kit a reagent required to specifically determine at least Tamm-Horsfall Urine Glycoprotein, optionally at least a further marker as described herein, e.g. Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2 and optionally auxiliary reagents for performing the determination.

Described is a kit comprising a reagent required to specifically determine at least Kidney-Injury-Molecule-1, optionally at least a further marker as described herein, e.g. Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2 and optionally auxiliary reagents for performing the determination.

Described is a kit comprising a reagent required to specifically determine at least Vascular Endothelial Growth Factor D, Nt-pro BNP, Growth-Factor-Differentiation-Factor-15 and Angiopoietin-2, and optionally auxiliary reagents for performing the determination.

Described is a kit comprising a reagent required to specifically determine at least Tamm-Horsfall Urine Glycoprotein, Nt-pro BNP, Growth-Factor-Differentiation-Factor-15 and Angiopoietin-2, and optionally auxiliary reagents for performing the determination.

Described is a kit comprising a reagent required to specifically determine at least Kidney-Injury-Molecule-1, Nt-pro BNP, Growth-Factor-Differentiation-Factor-15 and Angiopoietin-2, and optionally auxiliary reagents for performing the determination.

Described is a kit comprising a reagent required to specifically determine at least alpha-Glutathione-S-Transferase, Nt-pro BNP, Growth-Factor-Differentiation-Factor-15 and Angiopoietin-2, and optionally auxiliary reagents for performing the determination.

The invention also relates to a kit for performing the method according to the invention comprising a reagent required to specifically determine at least Vascular Endothelial Growth Factor D, Tamm-Horsfall Urine Glycoprotein, Kidney-Injury-Molecule-1, alpha-Glutathione-S-Transferase, Growth-Factor-Differentiation-Factor-15, Nt-pro BNP, and Angiopoietin-2 and optionally auxiliary reagents for performing the determination.

The invention also relates to a kit for performing the method according to the invention comprising a reagent required to specifically determine at least alpha-Glutathione-S-Transferase, Insulin-like Growth Factor Binding Protein 4 and Hepatocyte Growth Factor Receptor, and further markers from the group consisting of Nt-pro BNP, Angiopoietin-2, Growth-Factor-Differentiation-Factor-15, Apolipoprotein B, Osteoprotegerin and Chromogranin A, and auxiliary reagents for performing the determination.

Described is a kit comprising a reagent required to specifically determine at least Insulin-like Growth Factor Binding Protein 4 optionally at least a further marker as described herein, e.g. Nt-pro BNP, Peroxiredoxin-4, Osteoprotegerin, Apolipoprotein B, Apolipoprotein (a), Growth-Factor-Differentiation-Factor-15, Chromogranin A, Galectin-3, Tissue-Inhibitor of Metalloproteinases 1 and Angiopoietin-2 and optionally auxiliary reagents for performing the determination.

Described is a kit comprising a reagent required to specifically determine at least alpha-Glutathione-S-Transferase, Insulin-like Growth Factor Binding Protein 4, Chromogranin A, Hepatocyte Growth Factor Receptor, Nt-pro BNP, Angiopoietin-2, Growth-Factor-Differentiation-Factor-15, Apolipoprotein B, and Osteoprotegerin.

Described is a kit comprising a reagent required to specifically determine at least alpha-Glutathione-S-Transferase and/or Hepatocyte Growth Factor Receptor optionally at least a further marker and auxiliary reagents for performing the determination both as described herein.

Further markers may be selected from the group consisting of Nt-pro BNP, Angiopoietin-2, Growth-Factor-Differentiation-Factor-15, Apolipoprotein B, Osteoprotegerin and Chromogranin A.

Described is a kit comprising a reagent required to specifically determine at least alpha-Glutathione-S-Transferase, Chromogranin A, Hepatocyte Growth Factor Receptor, Nt-pro BNP, Angiopoietin-2, Growth-Factor-Differentiation-Factor-15, Apolipoprotein B, and Osteoprotegerin.

Described is a kit comprising a reagent required to specifically determine at least alpha-Glutathione-S-Transferase optionally at least a further marker and auxiliary reagents for performing the determination.

Further markers may be selected from the group consisting of Nt-pro BNP, Angiopoietin-2, Growth-Factor-Differentiation-Factor-15, Apolipoprotein B, Chromogranin A and Tissue-Inhibitor of Metalloproteinases-1.

Described is a kit for comprising a reagent required to specifically determine at least alpha-Glutathione-S-Transferase, Chromogranin A, Tissue Inhibitor of Metalloproteinases-1, Nt-pro BNP, Angiopoietin-2, Growth-Factor-Differentiation-Factor-15, and Apolipoprotein B.

Described is a kit comprising a reagent required to specifically determine at least Tamm-Horsfall Urine Glycoprotein optionally at least a further marker and auxiliary reagents for performing the determination.

Further markers may be selected from the group consisting of Nt-pro BNP, Growth-Factor-Differentiation-Factor-15, Apolipoprotein B, Chromogranin A, Tissue-Inhibitor of Metalloproteinases 1 and Apolipoprotein (a).

Described is a kit comprising a reagent required to specifically determine at least Tamm-Horsfall Urine Glycoprotein, Chromogranin A, Tissue Inhibitor of Metalloproteinases-1, Apolipoprotein (a), Nt-pro BNP, Growth-Factor-Differentiation-Factor-15 and Apolipoprotein B.

### Examples

### 1. Creation of a Model Building and Validation Set

As noted in the approved SAP, the 8401 individuals were divided into 2 groups stratified by region: a) model building (67%) and b) validation (33%) group. As no bloods were available from China, The regions were agreed upon as follows: North America and Australia; South America: Europe (including South Africa); and India. The characteristics of the participants in each set are noted below.

| **Table 1: All ORIGIN Participants** | | | | | | | |
|---|---|---|---|---|---|---|---|
| | | Overall | | Glargine | | Standard Care | |
| | N | N/Mean | %/SD | N/Mean | %/SD | N/Mean | %/SD |
| Categorical Variables | | | | | | | |
| --N. America + Australia | 12537 | 1516 | 12.1 | 762 | 12.2 | 754 | 12.0 |
| --S. America | 12537 | 3853 | 30.7 | 1925 | 30.7 | 1928 | 30.7 |
| --Europe | 12537 | 6060 | 48.3 | 3027 | 48.3 | 3033 | 48.4 |
| --India | 12537 | 390 | 3.1 | 194 | 3.1 | 196 | 3.1 |
| Prior CV Event* | 12533 | 7378 | 58.9 | 3712 | 59.3 | 3666 | 58.4 |
| Reported or measured Microalb/Alb* | 12537 | 3968 | 31.7 | 1984 | 31.7 | 1984 | 31.6 |
| Male | 12536 | 8150 | 65.0 | 4181 | 66.8 | 3969 | 63.3 |
| Male>=55y or female >=65y* | 12537 | 8765 | 69.9 | 4432 | 70.8 | 4333 | 69.1 |
| Current Smoking* | 12533 | 1552 | 12.4 | 781 | 12.5 | 771 | 12.3 |
| Prior diabetes* | 12536 | 10321 | 82.3 | 5162 | 82.4 | 5159 | 82.2 |
| Hypertension* | 12533 | 9963 | 79.5 | 4974 | 79.5 | 4989 | 79.5 |
| Age | 12537 | 63.05 | 7.82 | 63.05 | 7.79 | 63.04 | 7.85 |
| Continuous Variables | | | | | | | |
| --Cholesterol (mmol/L) | 12521 | 4.90 | 1.20 | 4.91 | 1.20 | 4.90 | 1.20 |
| --LDL Cholesterol (mmol/L)* | 12328 | 2.90 | 1.03 | 2.91 | 1.04 | 2.90 | 1.03 |
| --HDL Cholesterol (mmol/L) | 12471 | 1.19 | 0.32 | 1.19 | 0.32 | 1.20 | 0.32 |
| Outcome Variables | | | | | | | |
| Coprimary outcome 1 | 12537 | 2054 | 16.4 | 1041 | 16.6 | 1013 | 16.1 |
| Coprimary outcome 2 | 12537 | 3519 | 28.1 | 1792 | 28.6 | 1727 | 27.5 |
| Microvascular | 12537 | 2686 | 21.4 | 1323 | 21.1 | 1363 | 21.7 |
| New Diabetes | 12536 | 760 | 6.1 | 365 | 5.8 | 395 | 6.3 |
| Death | 12537 | 1916 | 15.3 | 951 | 15.2 | 965 | 15.4 |
| A1C <6% at 2 year visit | 12537 | 5729 | 45.7 | 3362 | 53.7 | 2367 | 37.7 |

| **Table 2: ORIGIN Biomarker Participants** | | | | | | | |
|---|---|---|---|---|---|---|---|
| | | Overall | | Glargine | | Standard Care | |
| | N | N/Mean | %/SD | N/Mean | %/SD | N/Mean | %/SD |
| Categorical Variables | | | | | | | |
| --N. America + Australia | 8401 | 1425 | 17.0 | 710 | 16.9 | 715 | 17.0 |
| --S. America | 8401 | 2772 | 33.0 | 1388 | 33.1 | 1384 | 32.9 |
| --Europe | 8401 | 3822 | 45.5 | 1903 | 45.4 | 1919 | 45.6 |
| --India | 8401 | 382 | 4.5 | 191 | 4.6 | 191 | 4.5 |
| Prior CV Event* | 8400 | 4991 | 59.4 | 2513 | 60.0 | 2478 | 58.9 |
| Reported or measured Microalb/Alb* | 8401 | 2656 | 31.6 | 1330 | 31.7 | 1326 | 31.5 |
| Male | 8401 | 5553 | 66.1 | 2834 | 67.6 | 2719 | 64.6 |
| Male>=55y or female >=65y* | 8401 | 5928 | 70.6 | 2997 | 71.5 | 2931 | 69.6 |
| Current Smoking* | 8400 | 1050 | 12.5 | 525 | 12.5 | 525 | 12.5 |
| Prior diabetes* | 8401 | 6840 | 81.4 | 3422 | 81.6 | 3418 | 81.2 |
| Hypertension* | 8400 | 6638 | 79.0 | 3320 | 79.2 | 3318 | 78.8 |
| Age | 8401 | 63.21 | 7.94 | 63.22 | 7.93 | 63.20 | 7.95 |
| Continuous Variables | | | | | | | |
| --Cholesterol (mmol/L) | 8393 | 4.89 | 1.18 | 4.89 | 1.17 | 4.89 | 1.18 |
| -LDL Cholesterol (mmol/L)* | 8278 | 2.90 | 1.03 | 2.90 | 1.03 | 2.89 | 1.02 |
| --HDL Cholesterol (mmol/L) | 8370 | 1.18 | 0.32 | 1.17 | 0.31 | 1.18 | 0.32 |
| Outcome Variables | | | | | | | |
| Coprimary outcome 1 | 8401 | 1405 | 16.7 | 727 | 17.3 | 678 | 16.1 |
| Coprimary outcome 2 | 8401 | 2435 | 29.0 | 1245 | 29.7 | 1190 | 28.3 |
| Microvascular | 8401 | 1794 | 21.4 | 887 | 21.2 | 907 | 21.5 |
| New Diabetes | 8401 | 550 | 6.5 | 259 | 6.2 | 291 | 6.9 |
| Death | 8401 | 1340 | 16.0 | 672 | 16.0 | 668 | 15.9 |
| A1C <6% at 2 year visit | 8401 | 4042 | 48.1 | 2389 | 57.0 | 1653 | 39.3 |

| **Table 3: Biomarker Participants in Model Building Group** | | | | | | | |
|---|---|---|---|---|---|---|---|
| | | Overall | | Glargine | | Standard Care | |
| | N | N/Mean | %/SD | N/Mean | %/SD | N/Mean | %/SD |
| Categorical Variables | | | | | | | |
| --N. America + Australia | 5630 | 955 | 17.0 | 491 | 17.3 | 464 | 16.6 |
| --S. America | 5630 | 1858 | 33.0 | 954 | 33.7 | 904 | 32.3 |
| --Europe | 5630 | 2561 | 45.5 | 1263 | 44.6 | 1298 | 46.4 |
| --India | 5630 | 256 | 4.5 | 123 | 4.3 | 133 | 4.8 |
| Prior CV Event* | 5630 | 3327 | 59.1 | 1680 | 59.3 | 1647 | 58.8 |
| Reported or measured Microalb/Alb* | 5630 | 1792 | 31.8 | 897 | 31.7 | 895 | 32.0 |
| Male | 5630 | 3680 | 65.4 | 1897 | 67.0 | 1783 | 63.7 |
| Male>=55y or female >=65y* | 5630 | 4003 | 71.1 | 2030 | 71.7 | 1973 | 70.5 |
| Current Smoking* | 5630 | 693 | 12.3 | 354 | 12.5 | 339 | 12.1 |
| Prior diabetes* | 5630 | 4590 | 81.5 | 2316 | 81.8 | 2274 | 81.2 |
| Hypertension* | 5630 | 4445 | 79.0 | 2242 | 79.2 | 2203 | 78.7 |
| Age | 5630 | 63.32 | 7.89 | 63.29 | 7.92 | 63.35 | 7.86 |
| Continuous Variables | | | | | | | |
| --Cholesterol (mmol/L) | 5623 | 4.90 | 1.19 | 4.91 | 1.18 | 4.89 | 1.21 |
| -LDL Cholesterol (mmol/L)* | 5550 | 2.91 | 1.03 | 2.92 | 1.04 | 2.89 | 1.03 |
| --HDL Cholesterol (mmol/L) | 5605 | 1.18 | 0.32 | 1.18 | 0.32 | 1.18 | 0.31 |
| Outcome Variables | | | | | | | |
| Coprimary outcome 1 | 5630 | 932 | 16.6 | 496 | 17.5 | 436 | 15.6 |
| Coprimary outcome 2 | 5630 | 1609 | 28.6 | 851 | 30.1 | 758 | 27.1 |
| Microvascular | 5630 | 1201 | 21.3 | 609 | 21.5 | 592 | 21.2 |
| New Diabetes | 5630 | 363 | 6.4 | 172 | 6.1 | 191 | 6.8 |
| Death | 5630 | 892 | 15.8 | 451 | 15.9 | 441 | 15.8 |
| A1C <6% at 2 year visit | 5630 | 2710 | 48.1 | 1626 | 57.4 | 1084 | 38.7 |

| **Table 4: Biomarker Participants in Validation Group** | | | | | | | |
|---|---|---|---|---|---|---|---|
| | | Overall | | Glargine | | Standard Care | |
| | N | N/Mean | %/SD | N/Mean | %/SD | N/Mean | %/SD |
| Categorical Variables | | | | | | | |
| --N. America + Australia | 2771 | 470 | 17.0 | 219 | 16.1 | 251 | 17.8 |
| --S. America | 2771 | 914 | 33.0 | 434 | 31.9 | 480 | 34.0 |
| --Europe | 2771 | 1261 | 45.5 | 640 | 47.0 | 621 | 44.0 |
| --India | 2771 | 126 | 4.5 | 68 | 5.0 | 58 | 4.1 |
| Prior CV Event* | 2770 | 1664 | 60.1 | 833 | 61.3 | 831 | 58.9 |
| Reported or measured Microalb/Alb* | 2771 | 864 | 31.2 | 433 | 31.8 | 431 | 30.6 |
| Male | 2771 | 1873 | 67.6 | 937 | 68.8 | 936 | 66.4 |
| Male>=55y or female >=65y* | 2771 | 1925 | 69.5 | 967 | 71.1 | 958 | 67.9 |
| Current Smoking* | 2770 | 357 | 12.9 | 171 | 12.6 | 186 | 13.2 |
| Prior diabetes* | 2771 | 2250 | 81.2 | 1106 | 81.3 | 1144 | 81.1 |
| Hypertension* | 2770 | 2193 | 79.2 | 1078 | 79.3 | 1115 | 79.1 |
| Age | 2771 | 63.00 | 8.04 | 63.08 | 7.96 | 62.92 | 8.12 |
| Continuous Variables | | | | | | | |
| --Cholesterol (mmol/L) | 2770 | 4.87 | 1.14 | 4.85 | 1.15 | 4.90 | 1.14 |
| -LDL Cholesterol (mmol/L)* | 2728 | 2.87 | 1.02 | 2.85 | 1.03 | 2.90 | 1.01 |
| --HDL Cholesterol (mmol/L) | 2765 | 1.18 | 0.31 | 1.16 | 0.30 | 1.19 | 0.33 |
| Outcome Variables | | | | | | | |
| Coprimary outcome 1 | 2771 | 473 | 17.1 | 231 | 17.0 | 242 | 17.2 |
| Coprimary outcome 2 | 2771 | 826 | 29.8 | 394 | 28.9 | 432 | 30.6 |
| Microvascular | 2771 | 593 | 21.4 | 278 | 20.4 | 315 | 22.3 |
| New Diabetes | 2771 | 187 | 6.7 | 87 | 6.4 | 100 | 7.1 |
| Death | 2771 | 448 | 16.2 | 221 | 16.2 | 227 | 16.1 |
| A1C <6% at 2 year visit | 2771 | 1332 | 48.1 | 763 | 56.1 | 569 | 40.4 |

### 2. Independent Predictors of the 1^{st} Co-Primary When Added to the Basic Clinical Model

1. Because the final biomarker list included 237 of the 284 biomarkers originally assayed, the p value for inclusion in the models in the SAP was increased from 0.05/284=0.00018 to 0.05/237 = 0.00021.
2. It was recognized that the sex and age criterion in the clinical model only adjusted for age (i.e. sex-specific age) by dichotomizing the age variable. Because sex was also considered important and may not have emerged well in the matched INTERHEART study, we agreed to adjust for sex in the model-building cohort. With respect to smoking, secondhand smoke was not included in the model as that data was not collected, and the smoking variable was simplified to current versus non-smoker.
3. The final variables forced into the basic clinical model before assessing any biomarkers were therefore the following (based on the SAP):

| | |
|---|---|
| a) Prior CV outcome (Y/N) | e) LDL cholesterol/HDL cholesterol |
| b) Reported/measured albuminuria (Y/N) | f) Current Smoker (Y/N) |
| c) Male ≥ 55 y or female ≥ 65 y (Y/N) | g) Prior Diabetes (Y/N) |
| d) Sex (M/F) | h) Prior Hypertension (Y/N) |

4. To assess the possibility that analyzing the ordinal and continuous data in the same model disadvantages the ordinal data which has 5 levels versus the continuous data which has infinite possible levels, a sensitivity analysis was run in which the raw, non-transformed data for every one of the 192 continuous variables was used to divide the data into 5ths using quintiles and the model was rerun. The results were compared to the model that mixed continuous and ordinal data.
a. When the ordinal and continuous biomarkers were included in the same analysis, these biomarkers were significant at P<0.05/237 when added to the clinical model:
   i. Trefoil Factor 3
   ii. Angiopoietin-2
   iii. N Terminal pro BNP (an ordinal biomarker)
   iv. alpha-Glutathione-S-Transferase
   v. Osteoprotegerin
   vi. Alpha-2-Macroglobulin
   vii. Peroxiredoxin-4
   viii. Apolipoprotein B
b. When the raw values of the continuous biomarkers were converted to 5 ordinal levels and added to the 45 ordinal biomarkers, these biomarkers were significant at P<0.05/237 when added to the clinical model (those biomarkers that differ from the ones identified in step a are noted in italics):
   *i*. Trefoil Factor 3
   ii. *VEGF D*
   iii. N Terminal pro BNP
   *iv.* alpha-Glutathione-S-Transferase
   v. *Growth*/*differentiation factor 15*
   vi. Alpha-2-Macroglobulin
   vii. Peroxiredoxin-4
   viii. Apolipoprotein B

5. Because one of the ordinal biomarkers made it into the model in step 4a (NT-pro BNP or var 190), a decision was made to use the model from step 4a since it used the most information to identify the included biomarkers. The model in step 4b will also be explored as a sensitivity analysis.
The final model derived from the model building set is noted below. No validation has yet been conducted in the validation set.

| **Cox Regression Identifying Independent Biomarker Predictors of the 1^{st} Co-Primary Outcome when 237 Biomarkers (192 Continuous & 45 Ordinal) were Added to the Basic Clinical Model using the Model Building Set** | | | | | | | | | |
|---|---|---|---|---|---|---|---|---|---|
| **Parameter** | **Label** | **Parameter Estimate** | **SE** | **SD** | **HR** | **Lower Bond** | **Upper Bond** | **P value** | **Inclusion p value** |
| pCV | Prior CV Event | 0.31373 | 0.07706 | . | 1.369 | 1.177 | 1.592 | 4.67E-05 | . |
| microalb | Albuminuria | -0.01641 | 0.07308 | . | 0.984 | 0.852 | 1.135 | 8.22E-01 | |
| SVSEX | Male | 0.4363 | 0.08453 | . | 1.547 | 1.311 | 1.826 | 2.45E-07 | . |
| gen age | M>=55, F>=65 | 0.14031 | 0.09826 | . | 1.151 | 0.949 | 1.395 | 1.53E-01 | . |
| LDL_HDL | LDL/HDL | 0.09881 | 0.0335 | 1.161 | 1.104 | 1.034 | 1.179 | 3.19E-03 | . |
| smk | Current Smoking | 0.37715 | 0.09364 | . | 1.458 | 1.214 | 1.752 | 5.63E-05 | . |
| pdiab | Prior diabetes | 0.10621 | 0.09072 | . | 1.112 | 0.931 | 1.328 | 2.42E-01 | . |
| hypten | Hypertension | 0.13973 | 0.08819 | . | 1.15 | 0.967 | 1.367 | 1.13E-01 | . |
| var262 | Trefoil Factor 3 | 0.28008 | 0.03536 | 0.108 | 1.323 | 1.235 | 1.418 | 2.33E-15 | 0.00E+00 |
| var190 | N-t pro BNP | 0.24933 | 0.03045 | 1.342 | 1.283 | 1.209 | 1.362 | 2.22E-16 | 0.00E+00 |
| var11 | Alpha-2-Macroglobulin | 0.1966 | 0.03723 | 2.678 | 1.217 | 1.132 | 1.309 | 1.29E-07 | 8.06E-12 |
| var207 | Peroxiredoxin-4 | 0.1458 | 0.03395 | 1.39 | 1.157 | 1.082 | 1.237 | 1.75E-05 | 3.53E-07 |
| var95 | alpha-Glutathione-S-Transferase | -0.17904 | 0.03457 | 31.193 | 0.836 | 0.781 | 0.895 | 2.24E-07 | 1.41E-05 |
| var199 | Osteo-protegerin | 0.14496 | 0.0384 | 2.252 | 1.156 | 1.072 | 1.246 | 1.60E-04 | 7.01 E-06 |
| var22 | Apolipoprotein B | 0.15647 | 0.03626 | 474.51 | 1.169 | 1.089 | 1.256 | 1.60E-05 | 3.58E-05 |
| var14 | Angiopoietin-2 | 0.14012 | 0.03571 | 3.681 | 1.15 | 1.073 | 1.234 | 8.73E-05 | 8.95E-05 |

P for inclusion < 0.05/237 or 0.00021097; Hazard ratios are expressed per 1 SD change in the parameter (NB - the SD is the SD of the transformed value if the variable was transformed because of non-normality)
**Likelihood Ratio Test:** Adding biomarkers to the basic clinical model increased the model chisquare from 198 to 613 (LR test =415 df=8 with p<0.001).
**C statistic with 95 %CI:** clinical model = 0.63(0.61, 0.65); clinical + biomarker = 0.72 (0.71, 0.74)
**Model calibration (Hosmer-Lemeshow) with 4 categories of risk rpobabilities (<5%, 5-10%, 10-20%, <20%):** Chisquare from 294 to 35 (p=5.58E-5) at max survival time (7.84 yrs) **Net reclassification Index (NRI)** using Bootstraps method =0.27(95% Cl 0.24, 0.32) at max survival time (7.84 yrs)
6. In order to explore the effect of the basic clinical model on the identity of the biomarkers included, the selection process was repeated after inclusion of a number of additional factors in the basic clinical model as noted below:

| **Biomarkers Included after Forcing creatininee &/or HbA1c into the Clinical Model** | | | | | | | | | |
|---|---|---|---|---|---|---|---|---|---|
| **Biomarker** | | **Clinical Model** | | **Clinical** + **Cr** | | **Clinical** + **HbA1c** | | **Clinical+ HbA1c & creatininee** | |
| | | HR | P | HR | P | HR | P | HR | P |
| var262 | Trefoil Factor 3 | 1.32 | 2.33E-15 | 1.234 | 2.88E-06 | 1.268 | 4.28E-09 | 1.217 | 1.22E-05 |
| var190 | NT pro BNP | 1.28 | 2.22E-16 | 1.29 | 1.23E-14 | 1.292 | 0.00E+00 | 1.312 | 2.22E-16 |
| var11 | Alpha 2 Macroglob | 1.2 | 1.29E-07 | X | | 1.211 | 3.87E-07 | X | |
| var207 | Peroxiredoxin-4 | 1.16 | 1.75E-05 | X | | 1.144 | 8.08E-05 | X | |
| var95 | Gluta-S-Transf alpha | 0.84 | 2.24E-07 | 0.844 | 9.13E-07 | 0.828 | 5.17E-08 | 0.837 | 3.21E-07 |
| var199 | Osteoprotegerin | 1.16 | 1.60E-04 | 1.21 | 7.11E-07 | X | | 1.179 | 2.12E-05 |
| var22 | Apolipoprotein B | 1.17 | 1.60E-05 | X | | 1.182 | 5.29E-06 | X | |
| var14 | Angiopoietin-2 | 1.15 | 8.73E-05 | 1.168 | 8.75E-06 | 1.147 | 1.09E-04 | 1.154 | 4.68E-05 |
| var101 | GDF 15 | X | | 1.21 | 3.43E-06 | 1.181 | 9.57E-05 | 1.193 | 2.93E-05 |
| var49 | Chromogranin A | X | | 0.841 | 1.36E-05 | X | | 0.836 | 8.00E-06 |
| var87 | Galectin-3 | X | | 0.878 | 2.01E-04 | X | | X | |
| C Stat | | 0.63(0.61, 0.65) 0.72 (0.71,0.74 | | 0.65 (0.63, 0.67) to 0.72 (0.70, 0.74) | | 0.64 (0.62, 0.65) to 0.72 (0.71, 0.74) | | 0.65(0.63, 0.67) to 0.72(0.70, 0.74) | |
| **Interpretation:** a) Both attenuate 262; b) creatininee eliminates var 11 and 207 & HbA1c no effect; c) HbA1c eliminates osteoprotegerin; d) creatininee eliminates Apo B; e) Both add GDF15; f) creatininee adds chromopranin A | | | | | | | | | |

| **Biomarkers Included after Forcing Drugs into the Clinical Model** | | | | | | | | | |
|---|---|---|---|---|---|---|---|---|---|
| **Biomarker** | | **Statin + Clinical** | | **ACE/ARB + Clinical** | | **ASA+ Clinical** | | **ACE/ARB, ASA, Statin** + **Clinical** | |
| | | **HR** | **P** | **HR** | **P** | **HR** | **P** | **HR** | **P** |
| var262 | Trefoil Factor 3 | 1.253 | 1.88E-08 | 1.323 | 2.55E-15 | 1.323 | 2.33E-15 | 1.253 | 1.92E-08 |
| var190 | NT pro BNP | 1.292 | 0.00E+00 | 1.282 | 3.33E-16 | 1.283 | 2.22E-16 | 1.292 | 0.00E+00 |
| var11 | Alpha 2 Macroglob | 1.206 | 5.42E-07 | 1.218 | 1.24E-07 | 1.218 | 1.26E-07 | 1.207 | 5.31E-07 |
| var207 | Peroxiredoxin-4 | 1.142 | 1.16E-04 | 1.157 | 1.70E-05 | 1.156 | 1.90E-05 | 1.142 | 1.16E-04 |
| var95 | Gluta-S-Transf alpha | 0.84 | 4.76E-07 | 0.836 | 2.17E-07 | 0.837 | 2.58E-07 | 0.84 | 4.95E-07 |
| var199 | Osteoprotegerin | | | 1.157 | 1.54E-04 | 1.156 | 1.66E-04 | | |
| var22 | Apolipoprotein B | 1.179 | 5.93E-06 | 1.17 | 1.52E-05 | 1.169 | 1.62E-05 | 1.179 | 5.85E-06 |
| var14 | Angiopoietin-2 | 1.152 | 6.60E-05 | 1.15 | 9.29E-05 | 1.15 | 9.09E-05 | 1.151 | 6.96E-05 |
| var101 | GDF 15 | 1.188 | 5.24E-05 | | | | | 1.187 | 5.36E-05 |
| var49 | Chromogranin A | | | | | | | | |
| C Stat | | 0.64(0.62, 0.65) - 0.72(0.71, 0.74) | | 0.63(0.61, 0.65) to 0.72(0.71, 0.74) | | 0.63(0.62, 0.65) to 0.72(0.71, 0.74) | | 0.64(0.62, 0.66) to 0.72(0.71, 0.74) | |

### 3. Independent Predictors of the 2^{nd} Co-Primary When Added to the Basic Clinical Model

1. Steps 1-3 from Section 2 are unchanged for this section
2. Step 4 from Section 2 was repeated and yielded the following results:
a. When the ordinal and continuous biomarkers were included in the same analysis, these biomarkers were significant at P<0.05/237 when added to the clinical model:
   i. N Terminal pro BNP (an ordinal biomarker)
   ii. Growth/differentiation factor 15 (GDF 15)
   iii. Angiopoietin-2
   iv. VEGF D (an ordinal biomarker)
   v. Tamm-Horsfall Urinary Glycoprotein
   vi. Kidney-Injury-Molecule-1(an ordinal biomarker)
   vii. Glutathione S-Transferase alpha
b. When the raw values of the continuous biomarkers were converted to 5 ordinal levels and added to the 45 ordinal biomarkers, the same biomarkers were significant at P<0.05/237 when added to the clinical model; the order of entry was slightly different however.
   i. N Terminal pro BNP
   ii. Growth/differentiation factor 15 (GDF 15)
   iii. VEGF D
   iv. Tamm-Horsfall Urinary Glycoprotein
   v. Kidney-Injury-Molecule-1
   vi. Glutathione S-Transferase alpha
   vii. Angiopoietin-2

3. The final model derived from the model building set is noted below. It satisfied the test for proportionality.

| C**ox Regression Identifying Independent Biomarker Predictors of the 2^{nd} Co-Primary Outcome when 237 Biomarkers (192 Continuous & 45 Ordinal) were Added to the Basic Clinical Model using the Model Building Set** | | | | | | | | | |
|---|---|---|---|---|---|---|---|---|---|
| **Parameter** | **Label** | **Parameter Estimate** | **SE** | **SD** | **HR** | **Lower Bond** | **Upper Bond** | **P value** | **Inclusion p value** |
| pCV | Prior CV Event | 0.4592 | 0.06001 | | 1.583 | 1.407 | 1.78 | 1.99E-14 | . |
| Microalb | Albuminuria | -0.00482 | 0.0573 | | 0.995 | 0.889 | 1.113 | 9.33E-01 | . |
| SVSEX | Male | 0.47127 | 0.06471 | | 1.602 | 1.411 | 1.819 | 3.28E-13 | . |
| gen_age | M>=55, F>=65 | 0.11315 | 0.07105 | | 1.12 | 0.974 | 1.287 | 1.11E-01 | . |
| LDL_HDL | LDL/HDL | 0.10632 | 0.02384 | 1.161 | 1.112 | 1.061 | 1.165 | 8.17E-06 | . |
| Smk | Current Smoking | 0.19299 | 0.07318 | | 1.213 | 1.051 | 1.4 | 8.36E-03 | . |
| Pdiab | Prior diabetes | 0.00283 | 0.06713 | | 1.003 | 0.879 | 1.144 | 9.66E-01 | . |
| hypten | Hypertension | 0.25264 | 0.06718 | | 1.287 | 1.129 | 1.469 | 1.69E-04 | . |
| var190 | NT-pro BNP | 0.16799 | 0.02209 | 1.342 | 1.183 | 1.133 | 1.235 | 2.90E-14 | 0.00E+00 |
| var101 | GDF15 | 0.14134 | 0.02931 | 0.42 | 1.152 | 1.088 | 1.22 | 1.41E-06 | 0.00E+00 |
| var14 | Angiopoietin-2 | 0.14823 | 0.02738 | 3.681 | 1.16 | 1.099 | 1.224 | 6.17E-08 | 1.67E-10 |
| var276 | VEGF D | 0.12275 | 0.02609 | 0.85 | 1.131 | 1.074 | 1.19 | 2.55E-06 | 2.18E-06 |
| var246 | Tamm-Horsfall Urine Glycoprotein | -0.11827 | 0.02817 | 0.019 | 0.888 | 0.841 | 0.939 | 2.69E-05 | 1.23E-05 |
| var152 | Kidne- Injury-Molecule-1 | 0.08215 | 0.01941 | 1.462 | 1.086 | 1.045 | 1.128 | 2.32E-05 | 1.12E-04 |
| var95 | Glutathione S-Transferase alpha | -0.10622 | 0.026 | 31.193 | 0.899 | 0.855 | 0.946 | 4.41E-05 | 4.37E-05 |

P for inclusion < 0.05/237 or 0.00021097; Hazard ratios are expressed per 1 SD change in the parameter (NB - the SD is the SD of the transformed value if the variable was transformed because of non-normality)
**Likelihood Ratio Test:** Adding biomarkers to the basic clinical model increased the model chisquare332 to 679 (LR test =348 df=7 with p<0.001).
**C statistic with 95 %CI:** clinical model = 0.63(0.61, 0.64); clinical + biomarker = 0.68 (0.67, 0.69)
**Model calibration (Hosmer-Lemeshow) with 4 categories of risk probabilities (<5%, 5-10%, 10-20%, <20%):** Chisquare from 110 to 18 (p<0.01) at max survival time (7.84 yrs)
**Net reclassification Index (NRI)** using Bootstraps method =0.12 (95% Cl 0.10, 0.14) at max survival time (7.84 y)

| **Biomarkers Predictive of Indicated Outcome After Accounting for Clinical Risk Factors** | | |
|---|---|---|
| **Based on the Model Buildinq Set** | | |
| **Biomarker** | **Co Primary 1** | **Co-Primary 2** |
| NT proBNP | X | X |
| Gluta S-Transferase alpha | X | X |
| GDF 15 | | X |
| Angiopoietin-2 | X | X |
| VEGF D | | X |
| Tamm-Horsfall Urine Gly-protein | | X |
| Kidney-Injury-Molecule-1 | | X |
| Trefoil Factor 3 | X | |
| Alpha-2-Macroglobulin | X | |
| Peroxiredoxin-4 | X | |
| Apolipoprotein B | X | |
| Osteoprotegerin | X | |

### 4. Validation of Results in the Validation Set

1. Using the validation set, a C statistic for both the clinical and clinical + biomarker models (identified in the model building sets), as well as the NRI were calculated for the 2 co-primary outcomes. Sensitivity and specificity for the cut-point that maximized these 2 values was also calculated. Forward selection was not done in the validation set. The following table shows the results of these analyses.

| | | **C stat (95% CI)** | **Cut-point** | **Sensitivity** | **Specificity** | **NRI(95% Cl)** |
|---|---|---|---|---|---|---|
| ***1^{st} Co-Primary Outcome*** | | | | | | |
| | Model Building: Clinical | 0.63(0.61- 0.65) | 50 | 0.67 | 0.54 | |
| | Model Building: Full | 0.72(0.71- 0.74) | 60 | 0.69 | 0.66 | 0.28(0.24- 0.32) |
| | Validation: Clinical | 0.65( 0.63- 0.68) | 55 | 0.64 | 0.6 | |
| | Validation: Full | 0.69( 0.67- 0.72) | 60 | 0.63 | 0.66 | 0.35( 0.29- 0.41) |

| ***2^{nd} Co-Primary Outcome*** | | | | | | |
|---|---|---|---|---|---|---|
| | Model Building: Clinical | 0.63( 0.62- 0.64) | 55 | 0.59 | 0.62 | |
| | Model Building: Full | 0.68( 0.67- 0.69) | 55 | 0.66 | 0.64 | 0.12( 0.10- 0.14) |
| | Validation: Clinical | 0.65( 0.63- 0.67) | 45 | 0.71 | 0.53 | |
| | Validation: Full | 0.68( 0.66- 0.70) | 65 | 0.53 | 0.73 | 0.04( 0.01- 0.06) |

| **Table 3: Model Performance of Participants in the ORIGIN Biomarker Study** | | | |
|---|---|---|---|
| | **Basic Clinical C (95%CI)** | **Full Model C (95% CI)** | **Improvement NRI (95%CI)** |
| ***1st Co-Primary Outcome*** | | | |
| Model Building Set | 0.63 (0.61, 0.65) | 0.72 (0.71, 0.74) | 0.28 (0.24, 0.32) |
| Validation Set* | 0.65 (0.63, 0.68) | 0.69 (0.67, 0.72) | 0.35 (0.29, 0.41) |
| All Participants | 0.64 (0.63, 0.65) | 0.72 (0.70, 0.73) | 0.25 (0.22, 0.28) |
| Nonfatal MI | 0.65 (0.62, 0.67) | 0.67 (0.64, 0.69) | 0.15 (0.09, 0.22) |
| Nonfatal Stroke | 0.64 (0.61, 0.66) | 0.69 (0.66, 0.71) | 0.23 (0.16, 0.30) |
| Cardiovascular Death | 0.65 (0.63, 0.67) | 0.78(0.76, 0.80) | 0.53 (0.48, 0.58) |

| ***2nd Co-Primary Outcome*** | | | |
|---|---|---|---|
| Model Building Set | 0.63 (0.62, 0.64) | 0.68 (0.67, 0.69) | 0.12 (0.10, 0.14) |
| Validation Set* | 0.65 (0.63, 0.67) | 0.68 (0.66, 0.70) | 0.04 (0.01, 0.06) |
| All Participants | 0.64 (0.62, 0.65) | 0.67 (0.66, 0.68) | 0.10 (0.08, 0.11) |

Conclusions from these analyses were that the validation set yielded findings consistent with the model building set.

### 5. Validation of Results in the entire Dataset

For the final validation the forward selection process was repeated with the full 8401 participants for the 1^{st} and 2^{nd} co-primary outcomes. These models, as well as estimates of hazard ratios using sensitivity analyses in which age, creatininee, and both age and creatininee are added to the basic clinical model are shown below for each outcome.

Also shown below for each outcome is an estimate of the HR and Cl for each of the biomarkers identified in the run of all 8401, as well as the C statistics and NRI for each model that was derived from running the clinical model 1000 times and the clinical + biomarker model 1000 times using bootstrapping (i.e. 1000 samples of 8401 randomly drawn with replacement).
a) **1st Co-primary Outcome**

| **Biomarkers for 1^{st} Co-Primary Detected with Forward Selection on 8401 Pts** | | | | | |
|---|---|---|---|---|---|
| **Parameter** | **Label** | **SD** | **HR** | **HR ICI** | **HR uCI** |
| pCV | Prior CV Event | . | 1.45 | 1.28 | 1.654 |
| microalb | Microalb/Alb | . | 1.07 | 0.95 | 1.21 |
| SVSEX | | . | 1.45 | 1.26 | 1.66 |
| gen age | M>=55y/F >=65y | . | 1.14 | 0.98 | 1.33 |
| LDL_HDL | LDL/HDL | 1.161 | 1.10 | 1.05 | 1.17 |
| smk | Current Smoking | . | 1.45 | 1.25 | 1.68 |
| pdiab | Prior diabetes | . | 1.13 | 0.98 | 1.31 |
| hypten | Hypertension | . | 1.18 | 1.03 | 1.37 |
| var262 | Trefoil Factor 3 | 0.108 | 1.22 | 1.14 | 1.30 |
| var190 | NT pro BNP | | 1.29 | 1.22 | 1.35 |
| var11 | Alpha-2-Macroglob | 2.678 | 1.15 | 1.08 | 1.22 |
| var14 | Angiopoietin-2 | 3.681 | 1.17 | 1.11 | 1.24 |
| var22 | Apolipoprotein B | 474.505 | 1.19 | 1.12 | 1.26 |
| var101 | Growth/Diff factor 15 | 0.42 | 1.19 | 1.11 | 1.28 |
| var95 | Glut S-Transferase alpha | 31.193 | 0.86 | 0.81 | 0.91 |
| var49 | Chromogranin A | 459.299 | 0.85 | 0.80 | 0.91 |
| var199 | Osteoprotegerin | 2.252 | 1.18 | 1.11 | 1.26 |
| var109 | Hep Grwth Fact Receptor | 17.397 | 0.89 | 0.85 | 0.95 |
| C statistic: from 0.64(0.63, 0.65) to 0.72 (0.70, 0.73); NRI with 95%CI: NRI=0.25(0.22, 0.28) | | | | | |

| **Sensitivity Analyses Based on Modifications of the Basic Clinical Model:1**^{st} **Co-Primary** | | | | | | |
|---|---|---|---|---|---|---|
| **Parameter** | **Label** | **SD** | **Model** | **Modified Basic Clinical Model** | | |
| | | | | **Age*** | **Cr** | **Age/Cr*** |
| pCV | Prior CV Event | . | 1.45 | 1.49 | 1.49 | 1.49 |
| microalb | Microalb/Alb | . | 1.07 | 1.09 | 1.06 | 1.08 |
| SVSEX | | . | 1.45 | 1.49 | 1.33 | 1.32 |
| gen age | M>=55y/F >=65y | . | 1.14 | N/A | 1.14 | N/A |
| Age | Sensitivity Age | 7.89 | N/A | 1.22* | N/A | 1.21* |
| LDL_HDL | LDL/HDL | 1.161 | 1.10 | 1.10 | 1.09 | 1.10 |
| smk | Current Smoking | . | 1.45 | 1.56 | 1.49 | 1.59 |
| pdiab | Prior diabetes | . | 1.13 | 1.13 | 1.13 | 1.16 |
| hypten | Hypertension | . | 1.18 | 1.20 | 1.17 | 1.18 |
| creatininee | Sensitivity creatininee | 22.303 | | N/A | 1.08 | 1.07 |
| var262 | Trefoil Factor 3 | 0.108 | 1.22 | 1.24 | 1.21 | 1.22 |
| var190 | NT pro BNP | | 1.29 | 1.28 | 1.30 | 1.27 |
| var11 | Alpha-2-Macroglob | 2.678 | 1.15 | 1.46 | 1.15 | 1.15 |
| var14 | Angiopoietin-2 | 3.681 | 1.17 | 1.15 | 1.15 | 1.16 |
| var22 | Apolipoprotein B | 474.505 | 1.19 | 1.20 | 1.18 | 1.19 |
| var101 | Growth/Diff factor 15 | 0.42 | 1.19 | 1.18 | 1.21 | 1.18 |
| var95 | Glut S-Transferase alpha | 31.193 | 0.86 | 0.88 | 0.85 | 0.87 |
| var49 | Chromogranin A | 459.299 | 0.85 | 0.88 | 0.86 | 0.88 |
| var199 | Osteoprotegerin | 2.252 | 1.18 | N/A | 1.16 | N/A |
| var109 | Hep Grwth Fact Receptor | 17.397 | 0.89 | N/A | N/A | N/A |
| Var207 | Peroxiredoxin-4 | 1.39 | N/A | 1.12 | 1.11 | 1.14 |
| Var87 | Galectin 3 | 4.034 | N/A | 0.91 | 0.88 | 0.89 |
| Var193 | Neuropilin 1 | 46.16 | N/A | N/A | 0.89 | N/A |
| *these models included an interaction term of age*time because the hazard for the age variable violated the assumptions of non-proportionality (supremum test=0.02); the HR for the age estimate is based on a time period of 5 years | | | | | | |

| **Biomarkers for 1^{st} Co-Primary Detected with Forward Selection on 8401 Pts; HRs & C statistics & NRI Estimated Using Bootstrap Techniques** | | | | | |
|---|---|---|---|---|---|
| **Parameter** | **Label** | **SD** | **HR** | **HR ICI** | **HR uCI** |
| pCV | Prior CV Event | . | 1.46 | 1.30 | 1.64 |
| microalb | Microalb/Alb | . | 1.07 | 0.95 | 1.21 |
| SVSEX | | . | 1.45 | 1.26 | 1.66 |
| gen age | M>=55y/F >=65y | . | 1.14 | 0.97 | 1.33 |
| LDL_HDL | LDL/HDL | 1.161 | 1.11 | 1.04 | 1.17 |
| smk | Current Smoking | . | 1.45 | 1.26 | 1.66 |
| pdiab | Prior diabetes | . | 1.14 | 0.99 | 1.31 |
| hypten | Hypertension | . | 1.19 | 1.01 | 1.39 |
| var262 | Trefoil Factor 3 | 0.108 | 1.22 | 1.15 | 1.30 |
| var190 | NT pro BNP | | 1.28 | 1.21 | 1.36 |
| var11 | Alpha-2-Macroglob | 2.678 | 1.15 | 1.08 | 1.22 |
| var14 | Angiopoietin-2 | 3.681 | 1.17 | 1.11 | 1.24 |
| var22 | Apolipoprotein B | 474.505 | 1.19 | 1.12 | 1.26 |
| var101 | Growth/Diff factor 15 | 0.42 | 1.19 | 1.10 | 1.28 |
| var95 | Glut S-Transferase alpha | 31.193 | 0.86 | 0.81 | 0.91 |
| var49 | Chromogranin A | 459.299 | 0.85 | 0.80 | 0.90 |
| var199 | Osteoprotegerin | 2.252 | 1.19 | 1.12 | 1.26 |
| var109 | Hep Grwth Fact Receptor | 17.397 | 0.90 | 0.84 | 0.95 |
| **C statistic: from 0.64(0.629, 0.656) to 0.72 (0.707, 0.733); NRI with 95%Cl: NRI=0.29(0.213, 0.406)** | | | | | |

| **Comparisons of Identified Biomarkers Using all 8401 vs. Model Building:** 1^{st} **Co-Primary** | | | |
|---|---|---|---|
| | | **Model Building (N=5630)** | **All Participants (N=8401)** |
| pCV | Prior CV Event | 1.37 | 1.45 |
| microalb | Microalb/Alb | 0.98 | 1.07 |
| SVSEX | | 1.55 | 1.45 |
| gen age | M>=55y/F >=65y | 1.15 | 1.14 |
| LDL_HDL | LDL/HDL | 1.10 | 1.10 |
| smk | Current Smoking | 1.46 | 1.45 |
| pdiab | Prior diabetes | 1.11 | 1.13 |
| hypten | Hypertension | 1.15 | 1.18 |
| var262 | Trefoil Factor 3 | 1.32 | 1.22 |
| var190 | NT pro BNP | 1.28 | 1.29 |
| var11 | Alpha-2-Macroglob | 1.22 | 1.15 |
| var14 | Angiopoietin-2 | 1.15 | 1.17 |
| var22 | Apolipoprotein B | 1.17 | 1.19 |
| var199 | Osteoprotegerin | 1.16 | 1.18 |
| var95 | Glut S-Transferase alpha | 0.84 | 0.86 |
| var207 | Peroxiredoxin-4 | 1.16 | X |
| var101 | Growth/Diff factor 15 | X | 1.19 |
| var49 | Chromogranin A | X | 0.85 |
| var109 | Hep Grwth Fact Receptor | X | 0.89 |

**b) 2^{nd} Co-Primary Outcome**

| **Biomarkers for 2^{nd} Co-Primary Detected with Forward Selection on 8401 Pts** | | | | | |
|---|---|---|---|---|---|
| **Parameter** | **Label** | **SD** | **HR** | **HR ICI** | **HR uCI** |
| pCV | Prior CV Event | . | 1.70 | 1.54 | 1.87 |
| microalb | Microalb/Alb | . | 1.07 | 0.98 | 1.17 |
| SVSEX | | . | 1.57 | 1.41 | 1.75 |
| gen age | M>=55y/F >=65y | . | 1.14 | 1.01 | 1.28 |
| LDL_HDL | LDL/HDL | 1.161 | 1.09 | 1.05 | 1.14 |
| smk | Current Smoking | . | 1.32 | 1.18 | 1.48 |
| pdiab | Prior diabetes | . | 1.05 | 0.94 | 1.17 |
| hypten | Hypertension | . | 1.31 | 1.18 | 1.46 |
| var190 | NT pro BNP | | 1.23 | 1.18 | 1.28 |
| var116 | IGF BP4 | 244.146 | 1.11 | 1.05 | 1.17 |
| var14 | Angiopoietin-2 | 3.681 | 1.19 | 1.13 | 1.24 |
| var101 | Growth/Diff factor 15 | 0.42 | 1.18 | 1.12 | 1.24 |
| var49 | Chromogranin A | 459.299 | 0.87 | 0.83 | 0.91 |
| var22 | Apolipoprotein B | 474.505 | 1.13 | 1.08 | 1.18 |
| var95 | Glut S-Transferase alpha | 31.193 | 0.92 | 0.88 | 0.96 |
| var199 | Osteoprotegerin | 2.252 | 1.17* | 1.10* | 1.24* |
| var109 | Hep Grwth Fact receptor | 17.397 | 0.91 | 0.88 | 0.95 |
| var199t | INTERACTION | | | | |
| *these models included an interaction term of var199*time because the hazard for var199 violated the assumptions of non-proportionality (supremum test=0.03); the HR for var199 is based on a time period of 5 years; | | | | | |
| C statistic at max follow-up (7.8 years) = 0.64 (0.62, 0.65) to 0.67 (0.66, 0.68); NRI at this time point =0.10 (0.08, 0.11) | | | | | |

| **Sensitivity Analyses Based on Modifications of the Basic Clinical Model:2^{nd} Co-Primary** | | | | | | |
|---|---|---|---|---|---|---|
| **Parameter** | **Label** | **SD** | **Model** | **Modified Basic Clinical Model** | | |
| | | | | **Age*** | **Cr**** | **Age/Cr*** |
| pCV | Prior CV Event | - | 1.70 | 1.69 | 1.69 | 1.71 |
| microalb | Microalb/Alb | - | 1.07 | 1.08 | 1.07 | 1.08 |
| SVSEX | | - | 1.57 | 1.65 | 1.46 | 1.53 |
| gen age | M>=55y/F >=65y | - | 1.14 | N/A | 1.15 | N/A |
| Age | Sensitivity Age | 7.89 | N/A | 1.20* | N/A | 1.18* |
| LDL_HDL | LDL/HDL | 1.161 | 1.09 | 1.09 | 1.09 | 1.08 |
| smk | Current Smoking | - | 1.32 | 1.38 | 1.35 | 1.39 |
| pdiab | Prior diabetes | - | 1.05 | 1.06 | 1.06 | 1.06 |
| hypten | Hypertension | - | 1.31 | 1.32 | 1.31 | 1.32 |
| creatininee | Sensitivity creatininee | 22.303 | N/A | N/A | 1.09 | 1.08 |
| var190 | NT pro BNP | | 1.23 | 1.23 | 1.23 | 1.23 |
| var116 | IGF BP4 | 244.146 | 1.11 | N/A | N/A | N/A |
| var14 | Angiopoietin-2 | 3.681 | 1.19 | N/A | 1.19 | 1.17 |
| var101 | Growth/Diff factor 15 | 0.42 | 1.18 | 1.19 | 1.19 | 1.18 |
| var49 | Chromogranin A | 459.299 | 0.87 | 0.85 | 0.85 | 0.85 |
| var22 | Apolipoprotein B | 474.505 | 1.13 | 1.10 | 1.13 | 1.12 |
| var95 | Glut S-Transferase alpha | 31.193 | 0.92 | N/A | 0.91 | 0.92 |
| var199 | Osteoprotegerin | 2.252 | 1.17* | N/A | 1.18* | N/A |
| var109 | Hep Grwth Fact receptor | 17.397 | 0.91 | N/A | 0.91 | N/A |
| var246 | T-H Urine Glycoprotein | 0.019 | N/A | 0.91 | N/A | N/A |
| var257 | Tissue Inhib M-proteinase1 | 40.073 | N/A | 1.09 | N/A | 1.10 |
| var28 | Apolipoprotein(a) | 395.331 | N/A | 1.09 | N/A | N/A |
| *these models included an interaction term of age*time because the hazard for the age variable violated the assumptions of non-proportionality (supremum test <0.001); the HR for the age estimate is based on a time period of 5 years; ** this model included an interaction term of var199*time because the hazard for var199 violated the assumptions of non-proportionality (supremum test =0.04); the HR for var199 is based on a time period of 5 years | | | | | | |

| **Biomarkers for 2^{nd} Co-Primary Detected with Forward Selection on 8401 Pts; HRs & C statistics & NRI Estimated Using Bootstrap Techniques** | | | | | |
|---|---|---|---|---|---|
| **Parameter** | **Label** | **SD** | **HR** | **HR ICI** | **HR uCI** |
| pCV | Prior CV Event | - | 1.70 | 1.54 | 1.87 |
| microalb | Microalb/Alb | - | 1.07 | 0.97 | 1.18 |
| SVSEX | | - | 1.58 | 1.41 | 1.78 |
| gen age | M>=55y/F >=65y | - | 1.14 | 1.01 | 1.28 |
| LDL_HDL | LDL/HDL | 1.161 | 1.09 | 1.05 | 1.14 |
| smk | Current Smoking | - | 1.32 | 1.18 | 1.49 |
| pdiab | Prior diabetes | - | 1.04 | 0.94 | 1.15 |
| hypten | Hypertension | - | 1.31 | 1.16 | 1.47 |
| var190 | NT pro BNP | | 1.23 | 1.19 | 1.28 |
| var116 | IGF BP4 | 244.146 | 1.11 | 1.04 | 1.17 |
| var14 | Angiopoietin-2 | 3.681 | 1.19 | 1.14 | 1.23 |
| var101 | Growth/Diff factor 15 | 0.42 | 1.17 | 1.11 | 1.24 |
| var49 | Chromogranin A | 459.299 | 0.87 | 0.82 | 0.92 |
| var22 | Apolipoprotein B | 474.505 | 1.13 | 1.08 | 1.17 |
| var95 | Glut S-Transferase alpha | 31.193 | 0.91 | 0.88 | 0.95 |
| var199 | Osteoprotegerin | 2.252 | 1.17* | 1.10* | 1.24* |
| var109 | Hep Grwth Fact receptor | 17.397 | 0.91 | 0.88 | 0.95 |
| var199t | INTERACTION | | | | |
| ***this HR is based on 5 yrs f/u; C 0.637(0.626, 0.648) to 0.686(0.676, 0.696); NRI=0.137(0.109, 0.173)** | | | | | |

| **Comparisons of Identified Biomarkers Using all 8401 vs. Model Building: 2^{nd} Co-Primary** | | | |
|---|---|---|---|
| | | **Model Building (N=5630)** | **All Participants (N=8401)** |
| pCV | Prior CV Event | 1.58 | 1.70 |
| microalb | Microalb/Alb | 1.00 | 1.07 |
| SVSEX | | 1.60 | 1.57 |
| gen age | M>=55y/F >=65y | 1.12 | 1.14 |
| LDL_HDL | LDL/HDL | 1.11 | 1.09 |
| smk | Current Smoking | 1.21 | 1.32 |
| pdiab | Prior diabetes | 1.00 | 1.05 |
| hypten | Hypertension | 1.29 | 1.31 |
| var190 | NT pro BNP | 1.18 | 1.23 |
| var14 | Angiopoietin-2 | 1.16 | 1.19 |
| var101 | Growth/Diff factor 15 | 1.15 | 1.18 |
| var95 | Glut S-Transferase alpha | 0.90 | 0.92 |
| Var246 | Tamm Hors Urin Glycoprot | 0.89 | X |
| Var152 | Kidne- Injury-Molecule-1 | 1.09 | X |
| Var276 | VEGF D | 1.13 | X |
| var22 | Apolipoprotein B | X | 1.13 |
| var199 | Osteoprotegerin | X | 1.15 |
| var116 | IGF BP4 | X | 1.17 |
| var49 | Chromogranin A | X | 0.87 |
| var109 | Hep Grwth Fact receptor | X | 0.91 |
| var199*t | INTERACTION | No | Yes |

## Claims

1. A method for assessing the risk for development of a cardiovascular disorder in a subject, comprising:
(a) determining in a sample from said subject
(i) the amount of the first markers Vascular Endothelial Growth Factor D, Tamm-Horsfall Urine Glycoprotein, Kidney-Injury-Molecule-1 and alpha-Glutathione-S-Transferase; and
(ii) the amount of the further markers Nt-pro BNP, Growth-Factor-Differentiation-Factor-15 and Angiopoietin-2; and
(b) correlating that said subject is at risk for development of a cardiovascular disorder when said amount is altered compared to a reference amount for the first and further markers.

2. A method for assessing the risk for development of a cardiovascular disorder in a subject, comprising:
(a) determining in a sample from said subject
(i) the amount of the first markers alpha-Glutathione-S-Transferase, Insulin-like Growth Factor Binding Protein-4 and Hepatocyte Growth Factor Receptor; and
(ii) the amount of the further markers Nt-pro BNP, Angiopoietin-2, Growth-Factor-Differentiation-Factor-15, Apolipoprotein B, Osteoprotegerin and Chromogranin A; and
(b) correlating that said subject is at risk for development of a cardiovascular disorder when said amount is altered compared to a reference amount for the first and further markers.

3. The method according to any one of the preceding claims wherein the cardiovascular disorder is myocardial infarction, stroke, and/or heart failure.

4. The method according to any one of the preceding claims wherein:
(i) the subject is pre-diabetic or diabetic;
(ii) the subject has an age of at least 50 years; and/or
(iii) the subject had a previous cardiovascular disorder.

5. The method according to claim 4 wherein the subject:
(i) is pre-diabetic or diabetic; and
(ii) has an age of at least 50 years; and
(iii) had a previous cardiovascular disorder.

6. The method according to any one of the preceding claims wherein the subject suffers from one or more of the risk factors selected from the group consisting of a previous cardiovascular disorder, albuminuria, male, age of at least 50 years, smoker, diabetic or pre-diabetic, elevated blood cholesterol, elevated blood creatinine, elevated HbA1c levels, obesity and hypertension.

7. The method of claim 6 wherein the subject suffers from the risk factors previous cardiovascular disorder, albuminuria, male, age of at least 55 years, elevated blood cholesterol level, smoker, pre-diabetic or diabetic and hypertension.

8. The method according to any one of the preceding claims wherein the sample is a body fluid and/or a tissue extract.

9. Use of Vascular Endothelial Growth Factor D, Tamm-Horsfall Urine Glycoprotein, Kidney-Injury-Molecule-1, alpha-Glutathione-S-Transferase, Nt-pro BNP, Growth-Factor Differentiation Factor-15 and Angiopoietin-2 in the *in vitro* assessment of the risk for development of a cardiovascular disorder in a subject, wherein determining an altered amount of said markers in a sample from the subject compared to a reference amount for said markers is indicative for said risk.

10. Use of alpha-Glutathione-S-Transferase, Insulin-like Growth Factor Binding Protein-4, Hepatocyte Growth Factor Receptor, Nt-pro BNP, Angiopoietin-2, Growth-Factor-Differentiation-Factor-15, Apolipoprotein B, Osteoprotegerin and Chromogranin A in the *in vitro* assessment of the risk for development of a cardiovascular disorder in a subject, wherein determining an altered amount of said markers in a sample from the subject compared to a reference amount for said markers is indicative for said risk.

11. A kit for performing the method according to any one of claims 1or 3-8 comprising a reagent required to specifically determine Vascular Endothelial Growth Factor D, Tamm-Horsfall Urine Glycoprotein, Kidney-Injury-Molecule-1, alpha-Glutathione-S-Transferase, Nt-pro BNP, Growth-Factor-Differentiation-Factor-15 and Angiopoietin-2, and optionally auxiliary reagents for performing the determination.

12. A kit for performing the method according to any one of items 2-8 comprising a reagent required to specifically determine alpha-Glutathione-S-Transferase, Insulin-like Growth Factor Binding Protein-4, Hepatocyte Growth Factor Receptor, Nt-pro BNP, Angiopoietin-2, Growth-Factor-Differentiation-Factor-15, Apolipoprotein B, Osteoprotegerin and Chromogranin A, and optionally reagents for performing the determination.

## Patentansprüche

1. Verfahren zur Beurteilung des Risikos der Entwicklung einer Herz-Kreislauf-Erkrankung bei einem Individuum, umfassend:
(a) Bestimmen, in einer Probe von dem Individuum,
(i) der Menge der ersten Marker VEGF (Vascular Endothelial Growth Factor) D, Tamm-Horsfall-Urin-Glycoprotein, KIM-1 (Kidney-Injury-Molecule-1) und Alpha-Glutathion-S-Transferase; und
(ii) der Menge der weiteren Marker Nt-pro-BNP, GDF-15 (Growth-Factor-Differentiation-Factor-15) und Angiopoetin-2; und
(b) Korrelieren, dass bei dem Individuum ein Risiko der Entwicklung einer Herz-Kreislauf-Erkrankung besteht, wenn die Menge verglichen mit einer Referenzmenge für die ersten und weiteren Marker verändert ist.

2. Verfahren zur Beurteilung des Risikos der Entwicklung einer Herz-Kreislauf-Erkrankung bei einem Individuum, umfassend:
(a) Bestimmen, in einer Probe von dem Individuum,
(i) der Menge der ersten Marker Alpha-Glutathion-S-Transferase, IGFBP-4 (Insulin-like Growth Factor Binding Protein-4) und HGFR (Hepatocyte Growth Factor Receptor); und
(ii) der Menge der weiteren Marker Nt-pro-BNP, Angiopoetin-2, GDF-15 (Growth-Factor-Differentiation-Factor-15), Apolipoprotein B, Osteoprotegerin und Chromogranin A; und
(b) Korrelieren, dass bei dem Individuum ein Risiko der Entwicklung einer Herz-Kreislauf-Erkrankung besteht, wenn die Menge verglichen mit einer Referenzmenge für die ersten und weiteren Marker verändert ist.

3. Verfahren nach einem der vorhergehenden Ansprüche, wobei es sich bei der Herz-Kreislauf-Erkrankung um Myokardinfarkt, Schlaganfall und/oder Herzinsuffizienz handelt.

4. Verfahren nach einem der vorhergehenden Ansprüche, wobei:
(i) das Individuum prädiabetisch oder diabetisch ist;
(ii) das Individuum wenigstens 50 Jahre alt ist; und/oder
(iii) das Individuum eine frühere Herz-Kreislauf-Erkrankung hatte.

5. Verfahren nach Anspruch 4, wobei das Individuum:
(i) prädiabetisch oder diabetisch ist; und
(ii) wenigstens 50 Jahre alt ist; und
(iii)eine frühere Herz-Kreislauf-Erkrankung hatte.

6. Verfahren nach einem der vorhergehenden Ansprüche, wobei das Individuum an einem oder mehreren der Risikofaktoren ausgewählt aus der Gruppe bestehend aus einer früheren Herz-Kreislauf-Erkrankung, Albuminurie, männlich, Alter von wenigstens 50 Jahren, Raucher, diabetisch oder prädiabetisch, erhöhtem Blutcholesterin, erhöhtem Blutkreatinin, erhöhten HbA1c-Spiegeln, Fettleibigkeit und Bluthochdruck leidet.

7. Verfahren gemäß Anspruch 6, wobei das Individuum an den Risikofaktoren frühere Herz-Kreislauf-Erkrankung, Albuminurie, männlich, Alter von wenigstens 55 Jahren, erhöhter Blutcholesterinspiegel, Raucher, prädiabetisch oder diabetisch und Bluthochdruck leidet.

8. Verfahren nach einem der vorhergehenden Ansprüche, wobei es sich bei der Probe um eine Körperflüssigkeit und/oder einen Gewebeextrakt handelt.

9. Verwendung von VEGF (Vascular Endothelial Growth Factor) D, Tamm-Horsfall-Urin-Glycoprotein, KIM-1 (Kidney-Injury-Molecule-1), Alpha-Glutathion-S-Transferase, Nt-pro-BNP, GDF-15 (Growth-Factor-Differentiation-Factor-15) und Angiopoetin-2 bei der In-vitro-Beurteilung des Risikos der Entwicklung einer Herz-Kreislauf-Erkrankung bei einem Individuum, wobei Bestimmen einer veränderten Menge der Marker in einer Probe von dem Individuum verglichen mit einer Referenzmenge für die Marker das Risiko anzeigt.

10. Verwendung von Alpha-Glutathion-S-Transferase, IGFBP-4 (Insulin-like Growth Factor Binding Protein-4), HGFR (Hepatocyte Growth Factor Receptor), Nt-pro-BNP, Angiopoetin-2, GDF-15 (Growth-Factor-Differentiation-Factor-15), Apolipoprotein B, Osteoprotegerin und Chromogranin A bei der In-vitro-Beurteilung des Risikos der Entwicklung einer Herz-Kreislauf-Erkrankung bei einem Individuum, wobei Bestimmen einer veränderten Menge der Marker in einer Probe von dem Individuum verglichen mit einer Referenzmenge für die Marker das Risiko anzeigt.

11. Kit zur Durchführung des Verfahrens nach einem der Ansprüche 1 oder 3-8, umfassend ein Reagens, das zur spezifischen Bestimmung von VEGF (Vascular Endothelial Growth Factor) D, Tamm-Horsfall-Urin-Glycoprotein, KIM-1 (Kidney-Injury-Molecule-1), Alpha-Glutathion-S-Transferase, Nt-pro-BNP, GDF-15 (Growth-Factor-Differentiation-Factor-15) und Angiopoetin-2 benötigt wird, und gegebenenfalls Hilfsreagentien zur Durchführung der Bestimmung.

12. Kit zur Durchführung des Verfahrens nach einem der Punkte 2-8, umfassend ein Reagens, das zur spezifischen Bestimmung von Alpha-Glutathion-S-Transferase, IGFBP-4 (Insulin-like Growth Factor Binding Protein-4), HGFR (Hepatocyte Growth Factor Receptor), Nt-pro-BNP, Angiopoetin-2, GDF-15 (Growth-Factor-Differentiation-Factor-15), Apolipoprotein B, Osteoprotegerin und Chromogranin A benötigt wird, und gegebenenfalls Reagentien zur Durchführung der Bestimmung.

## Revendications

1. Procédé pour évaluer le risque de développer un trouble cardio-vasculaire chez un sujet, comprenant les étapes consistant à :
(a) déterminer, dans un échantillon provenant dudit sujet,
(i) la quantité des premiers marqueurs facteur D de croissance endothéliale vasculaire, glycoprotéine urinaire de Tamm-Horsfall, molécule 1 d'atteinte rénale et alpha-glutathion-S transférase ; et
(ii) la quantité des marqueurs supplémentaires Nt-pro BNP, facteur 15 de différenciation d'un facteur de croissance et angiopoïétine-2 ; et
(b) établir une corrélation du fait que ledit sujet est à risque de développer un trouble cardio-vasculaire quand ladite quantité est altérée en comparaison avec une quantité de référence pour le premier marqueur et les marqueurs supplémentaires.

2. Procédé pour évaluer le risque de développer un trouble cardio-vasculaire chez un sujet, comprenant les étapes consistant à :
(a) déterminer, dans un échantillon provenant dudit sujet,
(i) la quantité des premiers marqueurs alpha-glutathion-S transférase, protéine 4 de liaison au facteur de croissance de type insuline et récepteur du facteur de croissance des hépatocytes ; et
(ii) la quantité des marqueurs supplémentaires Nt-pro BNP, angiopoïétine-2, facteur 15 de différenciation d'un facteur de croissance, apolipoprotéine B, ostéoprotégérine et chromogranine A ; et
(b) établir une corrélation du fait que ledit sujet est à risque de développer un trouble cardio-vasculaire quand ladite quantité est altérée en comparaison avec une quantité de référence pour le premier marqueur et les marqueurs supplémentaires.

3. Procédé selon l'une quelconque des revendications précédentes, dans lequel le trouble cardio-vasculaire est un infarctus du myocarde, un accident vasculaire cérébral et/ou la défaillance cardiaque.

4. Procédé selon l'une quelconque des revendications précédentes, dans lequel :
(i) le sujet est pré-diabétique ou diabétique ;
(ii) le sujet est âgé d'au moins 50 ans ; et/ou
(iii) le sujet a eu un trouble cardio-vasculaire préalable.

5. Procédé selon la revendication 4, dans lequel le sujet :
(i) est pré-diabétique ou diabétique ; et
(ii) est âgé d'au moins 50 ans ; et
(iii) a eu un trouble cardio-vasculaire préalable.

6. Procédé selon l'une quelconque des revendications précédentes, dans lequel le sujet a un ou plusieurs des facteurs de risque choisis dans le groupe constitué par un trouble cardio-vasculaire préalable, l'albuminurie, être un homme, être âgé d'au moins 50 ans, être fumeur, être diabétique ou pré-diabétique, un cholestérol sanguin élevé, une créatinine sanguine élevée, des taux de HbA1c élevés, l'obésité et l'hypertension.

7. Procédé de la revendication 6, dans lequel le sujet a les facteurs de risque : trouble cardio-vasculaire préalable, albuminurie, être un homme, être âgé d'au moins 55 ans, taux de cholestérol sanguin élevé, être fumeur, être pré-diabétique ou diabétique et hypertension.

8. Procédé selon l'une quelconque des revendications précédentes, dans lequel l'échantillon est un liquide corporel et/ou un extrait tissulaire.

9. Utilisation de facteur D de croissance endothéliale vasculaire, de glycoprotéine urinaire de Tamm-Horsfall, de molécule 1 d'atteinte rénale, d'alpha-glutathion-S transférase, de Nt-pro BNP, de facteur 15 de différenciation d'un facteur de croissance et d'angiopoïétine-2 dans l'évaluation *in vitro* du risque de développer un trouble cardio-vasculaire chez un sujet, dans laquelle une détermination, dans un échantillon provenant du sujet, d'une quantité altérée desdits marqueurs, en comparaison avec une quantité de référence pour lesdits marqueurs, est une indication dudit risque.

10. Utilisation d'alpha-glutathion-S transférase, de protéine 4 de liaison au facteur de croissance de type insuline, de récepteur du facteur de croissance des hépatocytes, de Nt-pro BNP, d'angiopoïétine-2, de facteur 15 de différenciation d'un facteur de croissance, d'apolipoprotéine B, d'ostéoprotégérine et de chromogranine A dans l'évaluation *in vitro* du risque de développer un trouble cardio-vasculaire chez un sujet, dans laquelle une détermination, dans un échantillon provenant du sujet, d'une quantité altérée desdits marqueurs, en comparaison avec une quantité de référence pour lesdits marqueurs, est une indication dudit risque.

11. Trousse pour exécuter le procédé selon l'une quelconque des revendications 1 ou 3 à 8, comprenant un réactif requis pour déterminer spécifiquement les : facteur D de croissance endothéliale vasculaire, glycoprotéine urinaire de Tamm-Horsfall, molécule 1 d'atteinte rénale, alpha-glutathion-S transférase, Nt-pro BNP, facteur 15 de différenciation d'un facteur de croissance et angiopoïétine-2 et, en option, des réactifs auxiliaires pour exécuter la détermination.

12. Trousse pour exécuter le procédé selon l'un quelconque des éléments 2 à 8, comprenant un réactif requis pour déterminer spécifiquement les : alpha-glutathion-S transférase, protéine 4 de liaison au facteur de croissance de type insuline, récepteur du facteur de croissance des hépatocytes, Nt-pro BNP, angiopoïétine-2, facteur 15 de différenciation d'un facteur de croissance, apolipoprotéine B, ostéoprotégérine et chromogranine A et, en option, des réactifs pour exécuter la détermination.
